# EUROPÄISCHE PATENTSCHRIFT

(11) **EP 1 713 811 B1**
(45) Veröffentlichungstag und Bekanntmachung des Hinweises auf die Patenterteilung: **01.10.2008**
(21) Anmeldenummer: 05700909.4
(22) Anmeldetag: 14.01.2005
(51) Int. Cl.: C07D 487/22, C09B 47/067, C10L 1/00, G01N 33/28

(54) **VERWENDUNG VON PHTHALOCYANINEN ALS MARKIERUNGSSTOFFE FÜR FLÜSSIGKEITEN**
USE OF PHTHALOCYANINES AS MARKING SUBSTANCES FOR LIQUIDS
PHTHALOCYANINES UTILISEES COMME MATIERES DE MARQUAGE POUR DES LIQUIDES

(30) Priorität: 23.01.2004 DE 102004003791
(43) Veröffentlichungstag der Anmeldung: 25.10.2006
(73) Patentinhaber: BASF SE, 67056 Ludwigshafen (DE)
(72) Erfinder: EBERT, Sophia, 68165 Mannheim (DE); GESSNER, Thomas, 69120 Heidelberg (DE); SENS, Rüdiger, 67069 Ludwigshafen (DE); VAMVAKARIS, Christos, 68163 Mannheim (DE)
(86) Internationale Anmeldenummer: PCT/EP2005/000307
(87) Internationale Veröffentlichungsnummer: WO 2005/070935

(56) Entgegenhaltungen:
- WO-A-94/02570
- WO-A-98/52950
- DE-B- 1 239 270
- JP-A- 2001 201 850
- US-A- 4 451 398

## Beschreibung

Die vorliegende Erfindung betrifft die Verwendung spezieller Phthalocyanine, welche über Methylengruppen gebundene Substituenten am Phthalocyaningrundgerüst tragen, als Markierungsstoffe für Flüssigkeiten, insbesondere Mineralöle, Flüssigkeiten, insbesondere Mineralöle, welche mindestens ein solches Phthalocyanin als Markierungsstoff enthalten, sowie neue spezielle Phthalocyanine, welche über Methylengruppen gebundene Substituenten am Phthalocyaningrundgerüst tragen.

Die Schrift EP 0 034 725 A2 beschreibt u.a. lmidazolylmethylgruppen enthaltende Phthalocyanine und deren Verwendung als nassechte Cellulosefarbstoffe.

Flockungsbeständige und lösungsmittelechte Phthalocyaninpigmentgemische, welche Phthalocyaninmethylenamine enthalten, werden in der deutschen Auslegeschrift 1 239 270 offenbart.

In der Schrift GB 2 328 184 A werden Pigmentzusammensetzungen mit u.a. hervorragenden rheologischen Eigenschaften beschrieben, welche aus der Additivierung mit Pigmentderivaten resultieren, die als Substituenten methylengebundene cyclische Reste aufweisen.

Dokument JP 2001-201850 A beschreibt Kupferphthalocyanine, die durch N-Methyl-1-piperidinylmethyl oder durch N-Methyl-4-Morpholinomethyl substituiert sind als Farbstoffe für photopolymerisierbare Bildaufzeichnungsmaterialien.

Die Verwendung der entsprechend substituierten Phthalocyanine als Markierungsstoffe für Flüssigkeiten, insbesondere Mineralöle, ist jedoch keiner der zuvor genannten Schriften zu entnehmen.

Als Markierungsstoffe für Flüssigkeiten, insbesondere Mineralöle, werden in der Schrift WO 94/02570 A1 neben anderen Verbindungen auch Phthalocyaninderivate vorge-schlagen.

Desweiteren werden in der Schrift WO 98/52950 A1 als Markierungsstoffe für Flüssigkeiten, insbesondere Mineralöle, Phthalocyanine beschrieben, welche als Substituenten fünf- oder sechsgliedrige gesättigte stickstoffhaltige heterocyclische Reste enthalten, die über ein Ringstickstoffatom an das Phthalocyaningrundgerüst gebunden sind.

Die Aufgabe der vorliegenden Erfindung bestand nun darin, Phthalocyanine zur Verfügung zu stellen, welche sich nicht nur durch gute Löslichkeit, sondern auch durch sehr gute Langzeitbeständigkeit in den zu markierenden Flüssigkeiten, insbesondere Mineralölen, auszeichnen.

Demgemäß wurde die Verwendung von Phthalocyaninen der Formel I 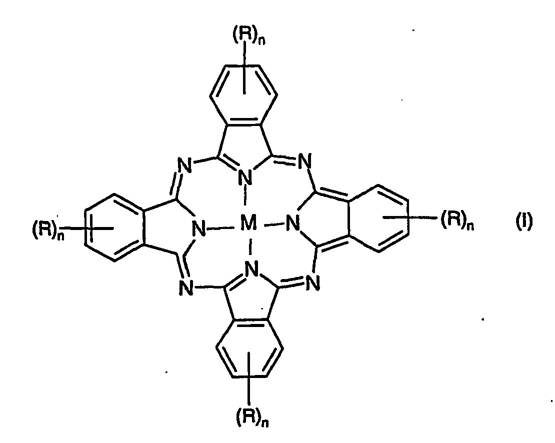 als Markierungsstoffe für Flüssigkeiten gefunden,
wobei in Formel I bedeuten
- M: zweimal Wasserstoff, zweimal Lithium, Magnesium, Zink, Kupfer, Nickel, VO, TiO, AlCl**,** AlOCOCH₃, AlOCOCF₃, SiCl₂ oder Si(OH)₂,
- R: gleiche oder voneinander verschiedene Gruppierungen ausgewählt aus der Gruppe bestehend aus -CH₂-N(-X¹-R¹)(-X²-R²) und -CH₂-Het,
- *X¹,* X²: unabhängig voneinander eine Carbonylgruppe oder eine chemische Einfach- bindung,
- R¹: C₁-C₂₀-Alkyl, das gegebenenfalls durch 1 bis 4 Sauerstoffatome in Etherfunk- tion unterbrochen ist,
C₅-C₇-Cycloalkyl, das gegebenenfalls mit einer oder mehreren C₁-C₂₀-Alkyl- gruppen, die gegebenenfalls durch 1 bis 4 Sauerstoffatome in Etherfunktion unterbrochen sind, substituiert ist,
gesättigter heterocyclischer fünf- oder sechsgliedriger Rest, der gegebenen- falls mit einer oder mehreren C₁-C₂₀-Alkylgruppen, die gegebenenfalls durch 1 bis 4 Sauerstoffatome in Etherfunktion unterbrochen sind, substituiert ist,
C₆-C₁₀-Aryl, das gegebenenfalls mit einem oder mehreren Halogen, Cyano, Nitro, Hydroxy, Amino, C₁-C₂₀-Alkyl, das gegebenenfalls durch 1 bis 4 Sauer- stoffatome in Etherfunktion unterbrochen ist, C₁-C₂₀-Alkoxy, C₁-C₂₀-Alkylamino oder C₁-C₂₀-Dialkylamino substituiert ist,
Heteroaryl mit 3 bis 12 Kohlenstoffatomen, das gegebenenfalls mit einem oder mehreren C₁-C₂₀-Alkyl, das gegebenenfalls durch 1 bis 4 Sauerstoffatome in Etherfunktion unterbrochen ist, C₁-C₂₀-Alk-oxy, C₁-C₂₀-Alkylamino oder C₁-C₂₀- Dialkylamino substituiert ist,
C₆-C₁₀-Aryl-C₁-C₄-alkyl, das im Arylrest gegebenenfalls mit einem oder mehre- ren Halogen, Cyano, Nitro, Hydroxy, Amino, C₁-C₂₀-Alkyl, das gegebenenfalls durch 1 bis 4 Sauerstoffatome in Etherfunktion unterbrochen ist, C₁-C₂₀-Al- koxy, C₁-C₂₀-Alkylamino oder C₁-C₂₀-Dialkylamino substituiert ist,
oder
Heteroaryl-C₁-C₄-alkyl mit 3 bis 12 Kohlenstoffatomen im Hetero-arylrest, wo- bei letzterer gegebenenfalls mit einem oder mehreren C₁-C₂₀-Alkyl, das gege- benenfalls durch 1 bis 4 Sauerstoffatome in Etherfunktion unterbrochen ist,
C₁-C₂₀-Alkoxy, C₁-C₂₀-Alkylamino oder C₁-C₂₀-Dialkylamino substituiert ist,
- R²: Wasserstoff oder unabhängig von R1 die Bedeutung von R1, wobei für den Fall, dass X² einer Carbonylgruppe entspricht, R² nicht die Bedeutung von Wasserstoff besitzt,
- Het: gesättigter heterocyclischer fünf-, sechs- oder siebengliedriger Rest, der ge- gebenenfalls mit einer oder mehreren C₁-C₂₀-Alkyl-gruppen, die gegebenen- falls durch 1 bis 4 Sauerstoffatome in Etherfunktion unterbrochen sind, substi- tuiert und in welchem gegebenenfalls eine CH₂-Gruppe durch eine Carbonyl- gruppe ersetzt ist,
oder
Heteroaryl mit 3 bis 12 Kohlenstoffatomen, das gegebenenfalls mit einer oder mehreren C₁-C₂₀-Alkylgruppen, die gegebenenfalls durch 1 bis 4 Sauerstoff- atome in Etherfunktion unterbrochen sind, substituiert ist,
wobei die Anbindung des gesättigten heterocyclischen Restes oder des Hete- roaryls an die CH₂-Gruppe der Gruppierung -CH₂-Het entweder über ein ge- eignetes Heteroatom oder ein Kohlenstoffatom erfolgt,
und
- n: jeweils unabhängig voneinander Werte von 0, 1, 2, 3 oder 4, mit der Maßga- be, dass die Summe der vier Werte von n mindestens 1 beträgt.

Als C₁-C₂₀-Alkyl, das gegebenenfalls durch 1 bis 4 Sauerstoffatome in Etherfunktion unterbrochen ist, sind zu nennen z.B. Methyl, Ethyl, Propyl, Isopropyl, Butyl, Isobutyl, sec-Butyl, tert-Butyl, Pentyl, Isopentyl, Neopentyl, tert-Pentyl, Hexyl, 2-Methylpentyl, Heptyl, Hept-3-yl, Octyl, 2-Ethylhexyl, Isooctyl, Nonyl, Isononyl, Decyl, Isodecyl, Undecyl, Dodecyl, Tridecyl, 3,5,5,7-Tetramethylnonyl, Isotridecyl (die obigen Bezeichnungen Isooctyl, Isononyl, Isodecyl und Isotridecyl sind Trivialbezeichnungen und stammen von den nach der Oxosynthese erhaltenen Alkoholen - vgl, dazu Ullmanns Encyklopädie der technischen Chemie, 4. Auflage, Band 7, Seiten 215 bis 217, sowie Band 11, Seiten 435 und 436), Tetradecyl, Pentadecyl, Hexadecyl, Heptadecyl, Octadecyl, Nonadecyl, Eicosyl, Methoxymethyl, 2-Ethylhexoxymethyl, 2-Methoxyethyl, 2-Ethoxyethyl, 2-Propoxyethyl, 2-Isopropoxyethyl, 2-Butoxyethyl, 2- oder 3-Methoxypropyl, 2- oder 3-Ethoxypropyl, 2- oder 3-Propoxypropyl, 2- oder 3-Butoxypropyl, 2- oder 4-Methoxybutyl, 2- oder 4-Ethoxybutyl, 2- oder 4-Propoxybutyl, 2- oder 4-Butoxybutyl, 3,6-Dioxaheptyl, 3,6-Dioxaoctyl, 4,8-Dioxanonyl, 3,7-Dioxaoctyl, 3,7-Dioxanonyl, 4,7-Dioxaoctyl, 4,7-Dioxanonyl, 4,8-Dioxadecyl, 3,6,8-Trioxadecyl, 3,6,9-Trioxaundecyl, 3,6,9,12-Tetraoxatridecyl oder 3,6,9,12-Tetraoxatetradecyl.

Als C₅-C₇-Cycloalkylreste kommen in Frage Cyclopentyl, Cyclohexyl und Cycloheptyl. Diese Cycloalkyle sind gegebenenfalls noch mit einer oder mehreren, insbesondere bis zu drei C₁-C₂₀-Alkylgruppen substituiert, wobei letztere gegebenenfalls noch durch 1 bis 4 Sauerstoffatome in Etherfunktion unterbrochen sind. Beispiele solcher gegebenenfalls noch durch Sauerstoffatome unterbrochene C₁-C₂₀-Alkylgruppen wurden bereits zuvor aufgeführt.

Gesättigte heterocyclische fünf- oder sechsgliedrige Reste, die gegebenenfalls mit einer oder mehreren C₁-C₂₀-Alkylgruppen, die gegebenenfalls durch 1 bis 4 Sauerstoffatome in Etherfunktion unterbrochen sind, substituiert sind, leiten sich beispielsweise ab von Pyrrolidin, 2- oder 3-Methylpyrrolidin, 2,4-Dimethyl-3-ethylpyrrolidin, Pyrazolidin, 2-, 3-, 4- oder 5-Methylpyrazolidin, Imidazolidin, 2-, 3-, 4- oder 5-Methylimidazolidin, Oxazolidin, 2-, 4- oder 5-Methyloxazolidin, Isoxazolidin, 3-, 4- oder 5-Methylisoxazolidin, Piperidin, 2-, 3-, 4-Methyl- oder -Ethylpiperidin, 2,6-Dimethylpiperidin, Piperazin, 4-(C₁-C₄-Alkyl)piperazin, wie 4-Methyl- oder 4-Ethylpiperazin, Morpholin, Thiomorpholin oder Thiomorpholin-S,S-dioxid. Die Terminologie "sich ableiten" ist hierbei dahingehend zu verstehen, dass man aus den zuvor beispielhaft genannten Heterocyclen die sich ableitenden Reste durch Abstrahieren eines kohlenstoff- oder, sofern vorhanden, heteroatomgebundenen Wasserstoffatoms erhält. Beispielsweise leiten sich vom Piperidin etwa die Reste Piperidin-1-yl und Piperidin-2-yl durch Abstrahieren des stickstoffgebundenen bzw. eines in 2-Position kohlenstoffgebundenen Wasserstoffatoms ab.

Sofern R¹ und R² einem gegebenenfalls substituierten gesättigten heterocyclischen fünf- oder sechsgliedrigen Rest entsprechen und sowohl X¹ als auch X² die Bedeutung einer chemischen Einfachbindung zukommt, erfolgt die Anbindung an das Stickstoffatom der Gruppierung -CH₂-N(-X¹-R¹)(-X²-R²) über ein Kohlenstoffatom des heterocyclischen fünf- oder sechsgliedrigen Restes.

Als C₆-C₁₀-Aryle sind insbesondere Phenyl und Naphthyl zu nennen. Diese sind gegebenenfalls mit einem oder mehreren Halogen, wie etwa Fluor, Chlor oder Brom, Cyano, Nitro, Hydroxy, Amino, C₁-C₂₀-Alkyl, das gegebenenfalls durch 1 bis 4 Sauerstoffatome in Etherfunktion unterbrochen ist, C₁-C₂₀-Alkoxy, C₁-C₂₀-Alkylamino oder C₁-C₂₀-Dialkyl-amino substituiert. Entsprechende C₁-C₂₀-Alkylreste, welche gegebenenfalls durch 1 bis 4 Sauerstoffatome in Etherfunktion unterbrochen sind, bzw. C₁-C₂₀-Alkylreste, welche in den C₁-C₂₀-Alkoxy-, C₁-C₂₀-Alkylamino- oder C₁-C₂₀-Dialkylaminogruppen enthalten sind, wurden exemplarisch bereits zuvor aufgeführt.

Heteroarylreste mit 3 bis 12 Kohlenstoffatomen, die gegebenenfalls mit einem oder mehreren C₁-C₂₀-Alkyl, das gegebenenfalls durch 1 bis 4 Sauerstoffatome in Etherfunktion unterbrochen ist, C₁-C₂₀-Alkoxy, C₁-C₂₀-Alkylamino oder C₁-C₂₀-Dialkylamino substituiert sind, leiten sich z.B. von Pyrrol, Furan, Thiophen, Pyrazol, Isoxazol, Isothiazol, Imidazol, 1 H-1,2,3-Triazol, 1 H-1,2,4-Triazol, Pyridin, Pyrazin, Pyridazin, 1 H-Azepin, 2H-Azepin, Oxazol, Thiazol, 1,2,3-, 1,2,4- oder 1,3,4-Oxadiazol, 1,2,3-, 1,2,4- oder 1,3,4-Thiadiazol sowie gegebenenfalls den benzo- oder dibenzoanellierten Ringen, wie z.B. Chinolin, Isochinolin, Indol, Benzo[b]furan (Cumaron), Benzo[b]thiophen (Thionaphthen), Carbazol, Dibenzofuran, Dibenzothiophen, 1 H-Indazol, Indoxazol, Benzo[d]isothiazol, Anthranil, Benzimidazol, Benzoxazol, Benzothiazol, Cinnolin, Phthalazin, Chinazolin, Chinoxalin oder Phenazin ab. C₁-C₂₀-Alkylsubstituenten, die gegebenenfalls durch 1 bis 4 Sauerstoffatome in Etherfunktion unterbrochen sind, wurden bereits zuvor exemplarisch genannt.

Sofern R¹ und R² einem gegebenenfalls substituierten Heteroaryl entsprechen und sowohl X¹ als auch X² die Bedeutung einer chemischen Einfachbindung zukommt, erfolgt die Anbindung an das Stickstoffatom der Gruppierung -CH₂-N(-X¹-R¹)(-X²-R²) über ein Kohlenstoffatom des Heteroaryls.

Als C₆-C₁₀Aryl-C₁-C₄-alkyle, welche im Arylrest gegebenenfalls mit einem oder mehreren Halogen, Cyano, Nitro, Hydroxy, Amino, C₁-C₂₀-Alkyl, das gegebenenfalls durch 1 bis 4 Sauerstoffatome in Etherfunktion unterbrochen ist, C₁-C₂₀-Alkoxy, C₁-C₂₀-Alkyl-amino oder C₁-C₂₀-Dialkylamino substituiert sind, sind insbesondere Benzyl, Phenylethyl, 3-Phenylpropyl und 4-Phenylbutyl zu nennen. Entsprechende C₁-C₂₀-Alkylreste, welche gegebenenfalls durch 1 bis 4 Sauerstoffatome in Etherfunktion unterbrochen sind, bzw. C₁-C₂₀-Alkylreste, welche in den C₁-C₂₀-Alkoxy-, C₁-C₂₀-Alkylamino- oder C₁-C₂₀-Dialkylaminogruppen enthalten sind, wurden exemplarisch bereits zuvor aufgeführt.

Heteroaryl-C₁-C₄-alkyle mit 3 bis 12 Kohlenstoffatomen im Heteroarylrest, wobei letzterer gegebenenfalls mit einem oder mehreren C₁-C₂₀-Alkyl, das gegebenenfalls durch 1 bis 4 Sauerstoffatome in Etherfunktion unterbrochen ist, C₁-C₂₀-Alkoxy, C₁-C₂₀-Alkylamino oder C₁-C₂₀-Dialkylamino substituiert ist, leiten sich beispielsweise von den bereits weiter oben genannten Heteroarylresten ab, welche an die C₁-C₄-Alkylreste entweder über ein Kohlenstoffatom oder ein zur Anbindung geeignetes Heteroatom des Heteroaryls angebunden sind. Entsprechende C₁-C₂₀-Alkylreste, welche gegebenenfalls durch 1 bis 4 Sauerstoffatome in Etherfunktion unterbrochen sind, bzw. C₁-C₂₀-Alkylreste, welche in den C₁-C₂₀-Alkoxy-, C₁-C₂₀-Alkylamino- oder C₁-C₂₀-Dialkylaminogruppen enthalten sind, wurden exemplarisch bereits zuvor aufgeführt.

Für die Variable Het kommen allgemein neben den bereits exemplarisch genannten gegebenenfalls substituierten gesättigten heterocyclischen fünf- oder sechsgliedrigen Resten, welche sich von den entsprechenden fünf- oder sechsgliedrigen Heterocyclen ableiten, zusätzlich als siebengliedrige Reste noch solche in Betracht, welche sich von den entsprechenden siebengliedrigen Heterocyclen ableiten. Als letztere seien beispielsweise Oxepan, Thiepan und Azepan genannt. Desweiteren kommen auch fünf-, sechs- oder siebengliedrige Reste in Frage, in welchen eine CH₂-Gruppe durch eine Carbonylgruppe ersetzt ist. Letztere sind insbesondere fünf-, sechs- oder siebengliedrige Lacton- oder Lactamreste.

Im Allgemeinen erfolgt die Anbindung des gesättigten heterocyclischen Restes oder des Heteroaryls an die CH₂-Gruppe der Gruppierung -CH₂-Het entweder über ein geeignetes Heteroatom oder ein Kohlenstoffatom.

Im Falle der zuvor genannten Lactame findet die Anbindung an die CH₂-Gruppe der Gruppierung -CH₂-Het vorzugsweise über das Ringstickstoffatom statt.

Bevorzugte Verwendung finden Phthalocyanine der Formel I, in welchen M zweimal Wasserstoff, zweimal Lithium, Magnesium, Zink, Kupfer, Nickel, VO, TiO, AlCl, AlOCOCH₃, AlOCOCF₃, SiCl₂ oder Si(OH)₂, R gleichen oder voneinander verschiedenen Gruppierungen -CH₂-Het und Het einem gesättigten heterocyclischen fünf-, sechs- oder siebengliedrigen Rest, welcher gegebenenfalls mit einer oder mehreren C₁-C₁₅-Alkylgruppen, die gegebenenfalls durch 1 bis 4 Sauerstoffatome in Etherfunktion unterbrochen sind, substituiert und in welchen gegebenenfalls eine CH₂-Gruppe durch eine Carbonylgruppe ersetzt ist, oder einem Heteroaryl mit 3 bis 5 Kohlenstoffatomen entspricht, welches gegebenenfalls mit einer oder mehreren C₁-C₁₅-Alkylgruppen, die gegebenenfalls durch 1 bis 4 Sauerstoffatome in Etherfunktion unterbrochen sind, substituiert ist.

Besonders bevorzugte Verwendung finden Phthalocyanine der Formel I, in welchen M zweimal Wasserstoff, zweimal Lithium, Magnesium, Zink, Kupfer, Nickel, VO, TiO, AlCl, AlOCOCH₃, AlOCOCF₃, SiCl₂ oder Si(OH)₂, R gleichen oder voneinander verschiedenen Gruppierungen -CH₂-Het und Het einem gesättigten heterocyclischen fünf-, sechs-oder siebengliedrigen Rest, der gegebenenfalls mit einer oder mehreren C₁-C₄-Alkylgruppen substituiert und in welchem gegebenenfalls eine CH₂-Gruppe durch eine Carbonylgruppe ersetzt ist, oder einem Heteroaryl mit 3 bis 5 Kohlenstoffatomen entspricht, das gegebenenfalls mit einer oder mehreren C₁-C₄-Alkylgruppen substituiert ist.

Besonders vorteilhaft finden Verwendung Phthalocyanine der Formel I, in welchen M zweimal Wasserstoff, R gleichen oder voneinander verschiedenen Gruppierungen -CH₂-Het und Het einem gesättigten stickstoffhaltigen fünf-, sechs- oder siebengliedrigen Rest, der gegebenenfalls mit einer oder mehreren C₁-C₄-Alkylgruppen substituiert und in welchem die dem Stickstoffatom benachbarte CH₂-Gruppe durch eine Carbonylgruppe ersetzt ist, oder einem stickstoffhaltigen Heteroaryl mit 3 bis 5 Kohlenstoffatomen entspricht, das gegebenenfalls mit einer oder mehreren C₁-C₄-Alkylgruppen substituiert ist.

In allen zuvor aufgeführten Bevorzugungen erfolgt in den Phthalocyaninen der Formel I die Anbindung des gesättigten heterocyclischen Restes oder des Heteroaryls an die CH₂-Gruppe der Gruppierung -CH₂-Het entweder über ein geeignetes Stickstoffatom oder ein Kohlenstoffatom; desweiteren nimmt in Formel I die Variable n vorzugsweise jeweils unabhängig voneinander Werte von 0, 1 oder 2 an, mit der Maßgabe, dass die Summe der vier Werte von n mindestens 1 beträgt.

Die im Rahmen der vorherigen Bevorzugungen genannten Reste für Het wurden bereits in der weiter oben gegebenenen Aufzählung exemplarisch mit aufgeführt.

Von besonderem Interesse ist die Verwendung von Phthalocyaninen der Formel I, in welchen M zweimal Wasserstoff, R gleichen Gruppierungen -CH₂-Het und Het einem γ-Butyrolactam-, δ-Valerolactam- oder ε-Caprolactamrest entspricht, der gegebenenfalls mit einer oder mehreren C₁-C₄-Alkylgruppen substituiert und an die CH₂-Gruppe der Gruppierung -CH₂-Het über das Stickstoffatom gebunden ist, wobei n jeweils unabhängig voneinander Werte von 0, 1 oder 2 annimmt, mit der Maßgabe, dass die Summe der vier Werte von n mindestens 1 beträgt.

Weitere bevorzugte Verwendung finden Phthalocyanine der Formel I, in welchen M zweimal Wasserstoff, zweimal Lithium, Magnesium, Zink, Kupfer, Nickel, VO, TiO, AlCl, AlOCOCH₃, AlOCOCF₃, SiCl₂ oder Si(OH)₂, R gleichen oder voneinander verschiedenen Gruppierungen -CH₂-N(-X¹-R¹)(-X²-R²), X¹ und X² unabhängig voneinander einer Carbonylgruppe oder einer chemische Einfachbindung, R¹einem C₁-C₁₅-Alkyl, das gegebenenfalls durch 1 bis 4 Sauerstoffatome in Etherfunktion unterbrochen ist, einem Cyclohexyl, das gegebenenfalls mit einer oder mehreren C₁-C₁₅-Alkylgruppen, die gegebenenfalls durch 1 bis 4 Sauerstoffatome in Etherfunktion unterbrochen sind, substituiert ist, einem gesättigten heterocyclischen fünf- oder sechsgliedrigen Rest, der gegebenenfalls mit einer oder mehreren C₁-C₁₅-Alkylgruppen, die gegebenenfalls durch 1 bis 4 Sauerstoffatome in Etherfunktion unterbrochen sind, substituiert ist, einem C₆-C₁₀-Aryl, das gegebenenfalls mit einem oder mehreren C₁-C₁₅-Alkyl, das gegebenenfalls durch 1 bis 4 Sauerstoffatome in Etherfunktion unterbrochen ist, C₁-C₁₅-Alkoxy, C₁-C₁₅-Alkylamino oder C₁-C₁₅-Dialkylamino substituiert ist, einem Heteroaryl mit 3 bis 5 Kohlenstoffatomen, das gegebenenfalls mit einem oder mehreren C₁-C₁₅-Alkyl, das gegebenenfalls durch 1 bis 4 Sauerstoffatome in Etherfunktion unterbrochen ist, C₁-C₁₅-Alkoxy, Q₁-C₁₅-Alkylamino oder C₁-C₁₅-Dialkylamino substituiert ist, einem Phenyl-C₁-C₄-alkyl, das im Arylrest gegebenenfalls mit einem oder mehreren C₁-C₁₅-Alkyl, das gegebenenfalls durch 1 bis 4 Sauerstoffatome in Etherfunktion unterbrochen ist, C₁-C₁₅-Alkoxy, C₁-C₁₅-Alkylamino oder C₁-C₁₅-Dialkylamino substituiert ist oder einem Heteroaryl-C₁-C₄-alkyl mit 3 bis 5 Kohlenstoffatomen im Heteroarylrest, wobei letzterer gegebenenfalls mit einem oder mehreren C₁-C₁₅-Alkyl, das gegebenenfalls durch 1 bis 4 Sauerstoffatome in Etherfunktion unterbrochen ist, C₁-C₁₅-Alkoxy, C₁-C₁₅-Alkylamino oder C₁-C₁₅-Dialkylamino substituiert ist, und R² Wasserstoff oder unabhängig von R¹ der Bedeutung von R¹ entspricht, wobei für den Fall, dass X² einer Carbonylgruppe entspricht, R² nicht die Bedeutung von Wasserstoff besitzt.

Besonders bevorzugte Verwendung finden Phthalocyanine der Formel I, in welchen M zweimal Wasserstoff, R gleichen oder voneinander verschiedenen Gruppierungen -CH₂-N(-X¹-R¹)(-X²-R²), X¹ und X² unabhängig voneinander einer Carbonylgruppe oder einer chemischen Einfachbindung, R¹ einem C₁-C₁₅-Alkyl, das gegebenenfalls durch 1 bis 4 Sauerstoffatome in Etherfunktion unterbrochen ist, einem Cyclohexyl, das gegebenenfalls mit einer oder mehreren C₁-C₁₅-Alkylgruppen, die gegebenenfalls durch 1 bis 4 Sauerstoffatome in Etherfunktion unterbrochen sind, substituiert ist, einem gesättigten heterocyclischen fünf- oder sechsgliedrigen Rest, der gegebenenfalls mit einer oder mehreren C₁-C₁₅-Alkylgruppen, die gegebenenfalls durch 1 bis 4 Sauerstoffatome in Etherfunktion unterbrochen sind, substituiert ist, einem Phenyl, das gegebenenfalls mit einem oder mehreren C₁-C₁₅-Alkyl, das gegebenenfalls durch 1 bis 4 Sauerstoffatome in Etherfunktion unterbrochen ist, C₁-C₁₅-Alkoxy, C₁-C₁₅-Alkylamino oder C₁-C₁₅-Dialkylamino substituiert ist, einem Phenyl-C₁-C₄-alkyl, das im Arylrest gegebenenfalls mit einem oder mehreren C₁-C₁₅-Alkyl, das gegebenenfalls durch 1 bis 4 Sauerstoffatome in Etherfunktion unterbrochen ist, C₁-C₁₅-Alkoxy, C₁-C₁₅-Alkylamino oder C₁-C₁₅-Dialkylamino substituiert ist, und R²Wasserstoff oder unabhängig von R¹ der Bedeutung von R¹ enstpricht, wobei für den Fall, dass X² einer Carbonylgruppe entspricht, R² nicht die Bedeutung von Wasserstoff besitzt.

In den zuvor aufgeführten Bevorzugungen nimmt die Variable n vorzugsweise jeweils unabhängig voneinander Werte von 0, 1 oder 2 an, mit der Maßgabe, dass die Summe der vier Werte von n mindestens 1 beträgt.

Die im Rahmen der vorherigen Bevorzugungen genannten Reste für R¹ und R² wurden bereits in der weiter oben gegebenenen Aufzählung exemplarisch mit aufgeführt.

Geeignete Flüssigkeiten, die erfindungsgemäß mittels der oben näher bezeichneten Phthalocyanine der Formel I und ihrer bevorzugten Ausführungsformen markiert werden können, sind insbesondere organische Flüssigkeiten, beispielsweise Alkohole, wie Methanol, Ethanol, Propanol, Isopropanol, Butanol, Isobutanol, sec-Butanol, Pentanol, Isopentanol, Neopentanol oder Hexanol, Glykole, wie 1,2-Ethylenglykol, 1,2- oder 1,3-Propylenglykol, 1,2-, 2,3- oder 1,4-Butylenglykol, Di- oder Triethylenglykol oder Di-oder Tripropylenglykol, Ether, wie Methyl-tertbutylether, 1,2-Ethylenglykolmono- oder - dimethylether, 1,2-Ethylenglykolmono- oder -diethylether, 3-Methoxypropanol, 3-lsopropoxypropanol, Tetrahydrofuran oder Dioxan, Ketone, wie Aceton, Methylethylketon oder Diacetonalkohol, Ester, wie Essigsäure- methylester, Essigsäureethylester, Essigsäurepropylester oder Essigsäurebutylester, aliphatische oder aromatische Kohlenwasserstoffe wie Pentan, Hexan, Heptan, Octan, Isooctan, Petrolether, Toluol, Xylol, Ethylbenzol, Tetralin, Dekalin, Dimethylnaphthalin, Testbenzin, Mineralöl, wie Benzin, Kerosin, Dieselöl oder Heizöl, natürliche Öle, wie Olivenöl, Sojaöl oder Sonnenblumenöl, oder natürliche oder synthetische Motoren-, Hydraulik- oder Getriebeöle, z.B. Fahrzeugmotorenöl oder Nähmaschinenöl, oder Bremsflüssigkeiten.

Besonders vorteilhaft verwendet man die obengenannten Phthalocyanine der Formel I und ihre bevorzugten Ausführungsformen zum Markieren von Mineralölen.

Gegenstand der vorliegenden Erfindung sind desweiteren Flüssigkeiten, welche mindestens ein Phthalocyanin der Formel I oder eine bevorzugte Ausführungsform davon als Markierungsstoff enthalten.

Insbesondere werden Mineralöle beansprucht, welche mindestens ein Phthalocyanin der Formel I oder eine bevorzugte Ausführungsform davon als Markierungsstoff enthalten.

Die Phthalocyanine der Formel I können auch als Komponente in Additiv-Konzentraten (im Folgenden, dem einschlägigen Sprachgebrauch folgend auch "Packages" genannt) zur Anwendung kommen, welche neben einem Trägeröl und einem Gemisch verschiedener Kraftstoffadditive in der Regel auch Farbstoffe sowie, zur unsichtbaren fiskalischen oder herstellerspezifischen Markierung, zusätzlich noch Markierungsstoffe enthalten. Diese Packages ermöglichen es, dass sich verschiedene Mineralölvertriebsfirmen aus einem "Pool" von unadditiviertem Mineralöl versorgen können und erst mit Hilfe ihrer individuellen Packages dem Mineralöl, z.B. während der Abfüllung in entsprechende Transportbehälter, die firmenspezifische Additivierung, Farbigkeit sowie Markierung verleihen.

Solche Packages enthalten dann als Komponenten insbesondere:
a) mindestens ein Phthalocyanin der Formel I oder dessen bevorzugte Ausführungsformen,
b) mindestens ein Trägeröl,
c) mindestens ein Additiv ausgewählt aus der Gruppe bestehend aus Detergenzien,
   Dispergatoren und
   ventilsitzverschleißhemmenden Additiven,
d) sowie gegebenenfalls weitere Additive und Hilfsmittel.

Als Trägeröle werden üblicherweise viskose, hochsiedende und insbesondere thermostabile Flüssigkeiten verwendet. Sie überziehen die heißen Metalloberflächen, z. B. die Einlaßventile, mit einem dünnen Flüssigkeitsfilm und verhindern oder verzögern dadurch die Bildung und Ablagerung von Zersetzungsprodukten an den Metalloberflächen.

Als Komponente b) der Kraft- und Schmierstoffadditiv-Konzentrate in Frage kommende Trägeröle sind beispielsweise mineralische Trägeröle (Grundöle), insbesondere solche der Viskositätsklasse "Solvent Neutral (SN) 500 bis 2000", synthetische Trägeröfe auf Basis von Olefinpolymerisaten mit M_{N} = 400 bis 1800, vor allem auf Polybuten- oder Polyisobuten-Basis (hydriert oder nicht hydriert), von Polyalphaolefinen oder Polyinternalolefinen sowie synthetische Trägeröle auf Basis alkoxylierter langkettiger Alkohole oder Phenole. Als Trägeröle zu verwendende Addukte von Ethylenoxid, Propylenoxid und/oder Butylenoxid an Polybutyl- oder Polyisobutenalkohole sind etwa in der Schrift EP 277 345 A1, weitere zu verwendende Polyalkenalkohol-Polyalkoxylate in der Schrift WO 00/50543 A1 beschrieben. Als weitere zu verwendende Trägeröle sind auch Polyalkenalkohol-Polyetheramine zu nennen, wie sie in der Schrift WO 00/61708 aufgeführt sind.

Selbstverständlich können auch Mischungen verschiedener Trägeröle zum Einsatz kommen, sofern sie untereinander und mit den übrigen Komponenten der Packages verträglich sind.

Vergaser und Einlaßsysteme von Verbrennungsmotoren, aber auch Einspritzsysteme für die Kraftstoffdosierung, werden in zunehmendem Maße durch Verunreinigungen belastet, die beispielsweise durch Staubteilchen aus der Luft und unverbrannte Kohlenwasserstoffreste aus dem Brennraum verursacht werden.

Zur Reduzierung oder Vermeidung dieser Verunreinigungen werden dem Kraftstoff Additive (detergenzien") zur Reinhaltung von Ventilen und Vergasern bzw. Einspritzsystemen beigegeben. Derartige Detergenzien gelangen im allgemeinen in Kombination mit einem oder mehreren Trägerölen zur Anwendung. Die Trägeröle üben eine zusätzliche "Waschfunktion" aus, unterstützen und fördern oft die Detergenzien in ihrer reinigenden und reinerhaltenden Wirkung und können so zur Reduzierung der benötigten Menge an Detergenzien beitragen.

Erwähnt sei hier weiter, dass viele der üblicherweise als Trägeröle verwendeten Substanzen zusätzliche Wirkung als Detergenzien und/oder Dispergatoren entfalten, weshalb in einem solchen Fall der Anteil an letzteren reduziert werden kann. Solche Trägeröle mit Detergens-/Dispergatorwirkung sind etwa in der letztgenannten WO-Schrift dargelegt.

Auch lässt sich die Wirkungsweise von Detergenzien, Dispergatoren und ventilsitzverschleißhemmenden Additiven oftmals nicht klar voneinander abgrenzen, weshalb diese Verbindungen summarisch unter Komponente c) aufgeführt sind. Übliche Detergenzien, welche in den Packages Anwendung finden, sind beispielsweise in den Schriften WO 00/50543 A1 und WO 00/61708 A1 aufgeführt und umfassen:
Polyisobutenamine, welche gemäß der EP-A 244 616 durch Hydroformylierung von hochreaktivem Polyisobuten und anschließende reduktive Aminierung mit Ammoniak, Monoaminen oder Polyaminen wie Dimethylenaminopropylamin, Ethylendiamin, Diethylentriamin, Triethylentetramin oder Tetraethylenpentamin erhältlich sind,
Poly(iso)butenamine, welche durch Chlorierung von Polybutenen oder Polyisobutenen mit Doppelbindungen überwiegend in der β- und γ-Position und anschließende Aminierung mit Ammoniak, Monoaminen oder den oben genannten Polyaminen erhältlich sind,
Poly(iso)butenamine, welche durch Oxidation von Doppelbindungen in Poly(iso)-butenen mit Luft oder Ozon zu Carbonyl- oder Carboxylverbindungen und anschließende Aminierung unter reduzierenden (hydrierenden) Bedingungen erhältlich sind,
Polyisobutenamine, welche gemäß der DE-A 196 20 262 aus Polyisobutenepoxiden durch Umsetzung mit Aminen und nachfolgende Dehydratisierung und Reduktion der Aminoalkohole erhältlich sind,
gegebenenfalls Hydroxylgruppen enthaltende Polyisobutenamine, welche gemäß der WO-A 97/03946 durch Umsetzung von Polyisobutenen mit einem mittleren Polymerisationsgrad P = 5 bis 100 mit Stickoxiden oder Gemischen aus Stickoxiden und Sauerstoff und anschließende Hydrierung dieser Umsetzungsprodukte erhältlich sind;
Hydroxylgruppen enthaltende Polyisobutenamine, welche gemäß der EP-A 476 485 durch Umsetzung von Polyisobutenepoxiden mit Ammoniak, Monoaminen oder den oben genannten Polyaminen erhältlich sind,
Polyetheramine, welche durch Umsetzung von C₂- bis C₃₀-Alkanolen, C₆- bis C₃₀-Alkandiolen, Mono- oder Di-C₂- bis C₃₀-Alkylaminen, C₁- bis C₃₀-Alkylcyclohexa-nolen oder C₁- bis C₃₀-Alkylphenolen mit 1 bis 30 mol Ethylenoxid und/oder Propylenoxid und/oder Butylenoxid pro Hydroxyl- bzw. Aminogruppe und anschließende reduktive Aminierung mit Ammoniak, Monoaminen oder den oben genannten Polyaminen erhältlich sind, sowie
"Polyisobuten-Mannichbasen", welche gemäß EP-A 831 141 durch Umsetzung von polyisobutensubstituierten Phenolen mit Aldehyden und Monoaminen oder den oben genannten Polyaminen erhältlich sind.

Weitere zu verwendende Detergenzien und/oder ventilsitzverschleißhemmende Additive sind beispielsweise in der Schrift WO 00/47698 A1 aufgeführt und umfassen Verbindungen, welche mindestens einen hydrophoben Kohlenwasserstoffrest mit einem zahlengemittelten Molekulargewicht (M_{N}) von 85 bis 20 000 und mindestens eine polare Gruppierung aufweisen, und welche ausgewählt sind aus:
(i) Mono- oder Polyaminogruppen mit bis zu 6 Stickstoffatomen, wobei mindestens ein Stickstoffatom basische Eigenschaften hat,
(ii) Nitrogruppen, ggf. in Kombination mit Hydroxylgruppen,
(iii) Hydroxylgruppen in Kombination mit Mono- oder Polyaminogruppen, wobei mindestens ein Stickstoffatom basische Eigenschaften hat,
(iv) Carboxylgruppen oder deren Alkalimetall- oder Erdalkalimetallsalzen,
(v) Sulfonsäuregruppen oder deren Alkalimetall- oder Erdalkalimetallsalzen,
(vi) Polyoxy-C₂- bis C₄-alkylengruppierungen, die durch Hydroxylgruppen, Mono- oder Polyaminogruppen, wobei mindestens ein Stickstoffatom basische Eigenschaften hat, oder durch Carbamatgruppen terminiert sind,
(vii) Carbonsäureestergruppen,
(viii) aus Bernsteinsäureanhydrid abgeleitete Gruppierungen mit Hydroxy-und/oder Amino- und/oder Amido- und/oder Imidogruppen und
(ix) durch Mannich-Umsetzung von phenolischen Hydroxylgruppen mit Aldehyden und Mono- oder Polyaminen erzeugte Gruppierungen.

Mono- oder Polyaminogruppen (i) enthaltende Additive sind vorzugsweise Polyalkenmono- oder Polyalkenpolyamine auf Basis von Polypropen oder von hochreaktivem (d.h. mit überwiegend endständigen Doppelbindungen - meist in der β- und γ-Position) oder konventionellem (d.h. mit überwiegend mittenständigen Doppelbindungen) Polybuten oder Polyisobuten mit M_{N} = 300 bis 5000. Derartige Additive auf Basis von hochreaktivem Polyisobuten, welche aus dem Polyisobuten, welches bis zu 20 Gew.-% n-Buten-Einheiten enthalten kann, durch Hydroformylierung und reduktive Aminierung mit Ammoniak, Monoaminen oder Polyaminen wie Dimethylaminopropylamin, Ethylendiamin, Diethylentriamin, Triethylentetramin oder Tetraethylenpentamin hergestellt werden können, sind insbesondere aus der Schrift EP 244 616 A2 bekannt. Geht man bei der Herstellung der Additive von Polybuten oder Polyisobuten mit überwiegend mittenständigen Doppelbindungen (meist in der β- und γ-Position) aus, bietet sich der Herstellweg durch Chlorierung und anschließende Aminierung oder durch Oxidation der Doppelbindung mit Luft oder Ozon zur Carbonyl- oder Carboxylverbindung und anschließende Aminierung unter reduktiven (hydrierenden) Bedingungen an. Zur Aminierung können hier die gleichen Amine wie oben für die reduktive Aminierung des hydroformylierten hochreaktiven Polyisobutens eingesetzt werden. Entsprechende Additive auf Basis von Polypropen sind insbesondere in der Schrift WO 94/24231 A1 beschrieben.

Weitere bevorzugte Monoaminogruppen (i) enthaltende Additive sind die Hydrierungsprodukte der Umsetzungsprodukte aus Polyisobutenen mit einem mittleren Polymerisationsgrad P = 5 bis 100 mit Stickoxiden oder Gemischen aus Stickoxiden und Sauerstoff, wie sie insbesondere in der Schrift WO 97/03946 A1 beschrieben sind.

Weitere bevorzugte Monoaminogruppen (i) enthaltende Additive sind die aus Polyisobutenepoxiden durch Umsetzung mit Aminen und nachfolgende Dehydratisierung und Reduktion der Aminoalkohole erhältlichen Verbindungen, wie sie insbesondere in der Schrift DE 196 20 262 A1 beschrieben sind.

Nitrogruppen, ggf. in Kombination mit Hydroxylgruppen, (ii) enthaltende Additive sind vorzugsweise Umsetzungsprodukte aus Polyisobutenen des mittleren Polymerisationsgrades P = 5 bis 100 oder 10 bis 100 mit Stickoxiden oder Gemischen aus Stickoxiden und Sauerstoff, wie sie insbesondere in den Schriften WO 96/03367 A1 und WO 96/03479 A1 beschrieben sind. Diese Umsetzungsprodukte stellen in der Regel Mischungen aus reinen Nitropolyisobutanen (z.B. α,β-Dinitropolyisobutan) und gemischten Hydroxynitropolyisobutanen (z.B. α-Nitro-β-hydroxypolyisobutan) dar.

Hydroxylgruppen in Kombination mit Mono- oder Polyaminogruppen (iii) enthaltende Additive sind insbesondere Umsetzungsprodukte von Polyisobutenepoxiden, erhältlich aus vorzugsweise überwiegend endständige Doppelbindungen aufweisendem Polyisobuten mit M_{N} = 300 bis 5000, mit Ammoniak, Mono- oder Polyaminen, wie sie insbesondere in der Schrift EP 476 485 A1 beschrieben sind.

Carboxylgruppen oder deren Alkalimetall- oder Erdalkalimetallsalze (iv) enthaltende Additive sind vorzugsweise Copolymere von C₂-C₄₀-Olefinen mit Maleinsäureanhydrid mit einer Gesamt Molmasse von 500 bis 20 000, deren Carboxylgruppen ganz oder teilweise zu den Alkalimetall- oder Erdalkalimetallsalzen und ein verbleibender Rest der Carboxylgruppen mit Alkoholen oder Aminen umgesetzt sind. Solche Additive sind insbesondere aus der Schrift EP 307 815 A1 bekannt. Derartige Additive dienen hauptsächlich zur Verhinderung von Ventilsitzverschleiß und können, wie in der Schrift WO 87/01126 A1 beschrieben, mit Vorteil in Kombination mit üblichen Detergenzien, wie Poly(iso)butenaminen oder Polyetheraminen, eingesetzt werden.

Sulfonsäuregruppen oder deren Alkalimetall- oder Erdalkalimetallsalze (v) enthaltende Additive sind vorzugsweise Alkalimetall- oder Erdalkalimetallsalze eines Sulfobernsteinsäurealkylesters, wie er insbesondere in der Schrift EP 639 632 A1 beschrieben ist. Derartige Additive dienen hauptsächlich zur Verhinderung von Ventilsitzverschleiß und können mit Vorteil in Kombination mit üblichen Detergenzien, wie Poly(iso)butenaminen oder Polyetheraminen, eingesetzt werden.

Polyoxy-C₂- bis C₄-alkylengruppierungen (vi) enthaltende Additive sind vorzugsweise Polyether oder Polyetheramine, welche durch Umsetzung von C₂- bis C₆₀-Alkanolen, C₆- bis C₃₀-Alkandiolen, Mono- oder Di-C₂-C₃₀-alkylaminen, C₁-C₃₀-Alkylcyclo-hexanolen oder C₁-C₃₀-Alkylphenolen mit 1 bis 30 mol Ethylenoxid und/oder Propylenoxid und/oder Butylenoxid pro Hydroxylgruppe oder Aminogruppe und, im Falle der Polyetheramine, durch anschließende reduktive Aminierung mit Ammoniak, Monoaminen oder Polyaminen erhältlich sind. Derartige Produkte werden insbesondere in den Schriften EP 310 875 A1, EP 356 725 A1, EP 700 985 A1 und US 4,877,416 beschrieben. Im Falle von Polyethern erfüllen solche Produkte auch Trägeröleigenschaften. Typische Beispiele hierfür sind Tridecanol- oder Isotridecanolbutoxylate, Isononylphenolbutoxylate sowie Polyisobutenolbutoxylate und -propoxylate sowie die entsprechenden Umsetzungsprodukte mit Ammoniak.

Carbonsäureestergruppen (vii) enthaltende Additive sind vorzugsweise Ester aus Mono-, Di- oder Tricarbonsäuren mit langkettigen Alkanolen oder Polyolen, insbesondere solche mit einer Mindestviskosität von 2 mm²/s bei 100°C, wie sie insbesondere in der Schrift DE 38 38 918 A1 beschrieben sind. Als Mono-, Di- oder Tricarbonsäuren können aliphatische oder aromatische Säuren eingesetzt werden, als Esteralkohole bzw.-polyole eignen sich vor allem langkettige Vertreter mit beispielsweise 6 bis 24 C-Atomen. Typische Vertreter der Ester sind Adipate, Phthalate, iso-Phthalate, Terephthalate und Trimellitate des iso-Octanols, iso-Nonanols, iso-Decanols und des iso-Tridecanols. Derartige Produkte erfüllen auch Trägeröleigenschaften.

Aus Bernsteinsäureanhydrid abgeleitete Gruppierungen mit Hydroxy- und/oder Amino- und/oder Amido- und/oder Imidogruppen (viii) enthaltende Additive sind vorzugsweise entsprechende Derivate von Polyisobutenylbernsteinsäureanhydrid, welche durch Umsetzung von konventionellem oder hochreaktivem Polyisobuten mit M_{N} = 300 bis 5000 mit Maleinsäureanhydrid auf thermischen Wege oder über das chlorierte Polyisobuten erhältlich sind. Von besonderem Interesse sind hierbei Derivate mit aliphatischen Polyaminen wie Ethylendiamin, Diethylentriamin, Triethylentetramin oder Tetraethylenpentamin. Derartige Ottokraftstoffadditive sind insbesondere in der Schrift US 4,849,572 beschrieben.

Durch Mannich-Umsetzung von phenolischen Hydroxylgruppen mit Aldehyden und Mono- oder Polyaminen erzeugte Gruppierungen (ix) enthaltende Additive sind vorzugsweise Umsetzungsprodukte von polyisobutensubstituierten Phenolen mit Formaldehyd und Mono- oder Polyaminen wie Ethylendiamin, Diethylentriamin, Triethylentetramin, Tetraethylenpentamin oder Dimethylaminopropylamin. Die polyisobutenylsubstituierten Phenole können aus konventionellem oder hochreaktivem Polyisobuten mit M_{N} = 300 bis 5000 stammen. Derartige "Polyisobuten-Mannichbasen" sind insbesondere in der Schrift EP 831 141 A1 beschrieben.

Zur genaueren Definition der einzelnen aufgeführten Additive wird hier auf die Offenbarungen der obengenannten Schriften des Standes der Technik ausdrücklich Bezug genommen.

Dispergatoren als Komponente c) sind beispielsweise Imide, Amide, Ester und Ammonium- und Alkalimetallsalze von Polyisobutenbemsteinsäureanhydriden. Diese Verbindungen finden insbesondere Einsatz in Schmierölen, teilweise jedoch auch als Detergenzien in Kraftstoffzusammensetzungen.

Weitere Additive und Hilfsmittel, welche gegebenenfalls als Komponente d) der Packages vorhanden sein können, sind
organische Lösungsmittel, z.B. Alkohole, wie Methanol, Ethanol, Propanol, Isopropanol, Butanol, Isobutanol, sec-Butanol, Pentanol, Isopentanol, Neopentanol oder Hexanol, z.B. Glykole, wie 1,2-Ethylenglykol, 1,2- oder 1,3-Propylenglykol, 1,2-, 2,3- oder 1,4-Butylenglykol, Di- oder Triethylenglykol oder Di- oder Tripropylenglykol, z.B. Ether, wie Methyltertbutylether, 1,2-Ethylenglykolmono- oder -dimethylether, 1,2-Ethylenglykolmono- oder -diethylether, 3-Methoxypropanol, 3-Isopropoxypropanol, Tetrahydrofuran oder Dioxan, z.B. Ketone, wie Aceton, Methylethylketon oder Diacetonalkohol, z.B. Ester, wie Essigsäuremethylester, Essigsäureethylester, Essigsäurepropylester oder Essigsäurebutylester, z.B. Laktame, wie N-Methylpyrrolidinon (NMP), z.B. aliphatische oder aromatische Kohlenwasserstoffe sowie deren Gemische, wie Pentan, Hexan, Heptan, Octan, Isooctan, Petrolether, Toluol, Xylol, Ethylbenzol, Tetralin, Dekalin, Dimethylnaphthalin oder Testbenzin und z.B. Mineralöl, wie Benzin, Kerosin, Dieselöl oder Heizöl,
Korrosionsinhibitoren, beispielsweise auf Basis von zur Filmbildung neigenden Ammoniumsalzen organischer Carbonsäuren oder von heterocyclischen Aromaten bei Buntmetallkorrosionsschutz,
Antioxidantien oder Stabilisatoren, beispielsweise auf Basis von Aminen wie p-Phenylendiamin, Dicyclohexylamin oder Derivaten hiervon oder von Phenolen wie 2,4-Di-tert.-butylphenol oder 3,5-Di-tert.-butyl-4-hydroxyphenylpropionsäure,
Demulgatoren,
Antistatikmittel,
Metallocene wie Ferrocen oder Methylcyclopentadienylmangantricarbonyl,
Schmierfähigkeitsverbesserer (Lubricity-Additive) wie bestimmte Fettsäuren, Alkenylbernsteinsäureester, Bis(hydroxyalkyl)fettamine, Hydroxyacetamide oder Ricinusöl,
Amine zur Absenkung des pH-Wertes des Kraftstoffes,
weitere, von Phthalocyaninen der Formel I und deren bevorzugten Ausführungsformen verschiedene Markierungsstoffe sowie
Farbstoffe.

Die Konzentration der Komponente a), d.h. des mindestens einen Phthalocyanins der Formel I oder dessen bevorzugten Ausführungsformen, in den Packages wird üblicherweise in einer solchen Höhe gewählt, dass nach Zugabe des Packages zum Mineralöl die gewünschte Konzentration an Markierungsstoff(en) darin enthalten ist. Übliche Konzentrationen der Markierungsstoffe im Mineralöl liegen etwa im Bereich von 0,01 bis zu einigen 10 Gew.-ppm.

Komponente b), d.h. das mindestens eine Trägeröl, ist in den Packages üblicherweise in einer Konzentration von 1 bis 50, insbesondere 5 bis 30 Gew.%, und Komponente c), d.h. das mindestens eine Detergens und/oder der mindestens eine Dispergator, üblicherweise in einer Konzentration von 25 bis 90 Gew.%, insbesondere 30 bis 80 Gew.%, enthalten, jeweils bezogen auf die Gesamtmenge der Komponenten a) bis c) und gegebenenfalls d), wobei die Summe der Einzelkonzentrationen der Komponenten a) bis c) und gegebenenfalls d) sich zu 100 Gew.% ergänzt.

Sofern als Komponente d) Korrosionsinhibitoren, Antioxidantien oder Stabilisatoren, Demulgatoren, Antistatikmittel, Metallocene, Schmierfähigkeitsverbesserer und Amine zur Absenkung des pH-Wertes des Kraftstoffes, in den Packages enthalten sind, beläuft sich deren Konzentration in der Summe üblicherweise auf nicht mehr als 10 Gew.%, bezogen auf die Gesamtmenge des Packages (d.h. die Gesamtmenge der Komponenten a) bis c) und d)), wobei die Konzentration der Korrosionsinhibitoren und Demulgatoren üblicherweise im Bereich von jeweils etwa 0,01 bis 0,5 Gew.-% der Gesamtmenge des Packages liegt.

Sofern als Komponente d) zusätzliche (d.h. nicht bereits mit den übrigen Komponenten eingebrachte) organische Lösungsmittel in den Packages enthalten sind, beläuft sich deren Konzentration in der Summe üblicherweise auf nicht mehr als 20 Gew.%, bezogen auf die Gesamtmenge des Packages. Diese Lösungsmittel stammen in der Regel von Lösungen der Markierungsstoffe und/oder Farbstoffe, welche im Hinblick auf eine genauere Dosierbarkeit -anstelle der reinen Markierungsstoffe und/oder Farbstoffeden Packages zugegeben werden.

Sofern als Komponente d) weitere, von Phthalocyaninen der Formel I oder deren bevorzugten Ausführungsformen verschiedene Markierungsstoffe in den Packages enthalten sind, bemisst sich deren Konzentration wiederum am Gehalt, welchen diese nach Zugabe der Packages im Mineralöl aufweisen sollen. Sinngemäß gilt das zu Komponente a) Gesagte.

Sofern als Komponente d) Farbstoffe in den erfindungsgemäßen Packages enthalten sind, liegt deren Konzentration üblicherweise etwa zwischen 0,1 bis 5 Gew.%, bezogen auf die Gesamtmenge des Packages.

Im Rahmen der vorliegenden Erfindung werden desweiteren Phthalocyanine der Formel la 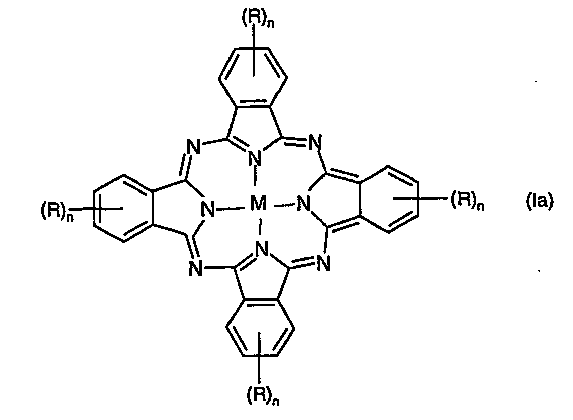 beansprucht, in welchen M zweimal Wasserstoff, zweimal Lithium, Magnesium, Zink, Kupfer, Nickel, VO, TiO, AlCl, AlOCOCH₃, AlOCOCF₃, SiCl₂ oder Si(OH)₂, R gleichen oder voneinander verschiedenen Gruppierungen -CH₂-Het und Het einem gesättigten heterocyclischen fünf-, sechs- oder siebengliedrigen Rest entspricht, der gegebenenfalls mit einer oder mehreren C₁-C₂₀-Alkylgruppen, die gegebenenfalls durch 1 bis 4 Sauerstoffatome in Etherfunktion unterbrochen sind, substituiert und in welchem gegebenenfalls eine CH₂-Gruppe durch eine Carbonylgruppe ersetzt ist, wobei die Anbindung des gesättigten heterocyclischen Restes an die CH₂-Gruppe der Gruppierung -CH₂-Het entweder über ein geeignetes Heteroatom oder ein Kohlenstoffatom erfolgt. Die Variable n nimmt jeweils unabhängig voneinander Werte von 0, 1, 2, 3 oder 4 an, mit der Maßgabe, dass die Summe der vier Werte von n mindestens 1 beträgt.

In den bevorzugten Phthalocyaninen der Formel I bedeutet M zweimal Wasserstoff, zweimal Lithium, Magnesium, Zink, Kupfer, Nickel, VO, TiO, AlCl, AlOCOCH₃, AIO-COCF₃, SiCl₂ oder Si(OH)₂, R gleiche oder voneinander verschiedene Gruppierungen -CH₂-Het und Het ein gesättigter heterocyclischer fünf-, sechs- oder siebengliedriger Rest, der gegebenenfalls mit einer oder mehreren C₁-C₁₅-Alkylgruppen, die gegebenenfalls durch 1 bis 4 Sauerstoffatome in Etherfunktion unterbrochen sind, substituiert und in welchem gegebenenfalls eine CH₂-Gruppe durch eine Carbonylgruppe ersetzt ist.

Besonders bevorzugte Phthalocyanine sind solche, in welchen in Formel la bedeutet M zweimal Wasserstoff, zweimal Lithium, Magnesium, Zink, Kupfer, Nickel, VO, TiO, AlCl, AlOCOCH₃, AlOCOCF₃, SiCl₂ oder Si(OH)₂, R gleiche oder voneinander verschiedene Gruppierungen -CH₂-Het und Het ein gesättigter heterocyclischer fünf-, sechs- oder siebengliedriger Rest, der gegebenenfalls mit einer oder mehreren C₁-C₄-Alkylgruppen substituiert und in welchem gegebenenfalls eine CH₂-Gruppe durch eine Carbonylgruppe ersetzt ist.

Von besonderem Interesse sind Phthalocyanine, in welchen in Formel la bedeutet M zweimal Wasserstoff, R gleiche oder voneinander verschiedene Gruppierungen -CH₂-Het und Het ein gesättigter stickstoffhaltiger fünf-, sechs- oder siebengliedriger Rest, der gegebenenfalls mit einer oder mehreren C₁-C₄-Alkylgruppen substituiert und in welchem die dem Stickstoffatom benachbarte CH₂-Gruppe durch eine Carbonylgruppe ersetzt ist.

In den zuvor aufgeführten Bevorzugungen erfolgt in den Phthalocyaninen der Formel la die Anbindung des gesättigten heterocyclischen Restes an die CH₂-Gruppe der Gruppierung -CH₂-Het entweder über ein geeignetes Stickstoffatom oder ein Kohlenstoffatom; desweiteren nimmt in Formel la die Variable n vorzugsweise jeweils unabhängig voneinander Werte von 0, 1 oder 2 an, mit der Maßgabe, dass die Summe der vier Werte von n mindestens 1 beträgt.

Von ganz besonderem Interesse sind Phthalocyanine, in welchen in Formel la bedeutet M zweimal Wasserstoff, R gleiche Gruppierungen -CH₂-Het, Het ein γ-Butyrolactam-, δ-Valerolactam- oder ε-Caprolactamrest, der gegebenenfalls mit einer oder mehreren C₁-C₄-Alkylgruppen substituiert und an die CH₂-Gruppe der Gruppierung -CH₂-Het über das Stickstoffatom gebunden ist, und n nimmt jeweils unabhängig voneinander Werte von 0, 1 oder 2 an, mit der Maßgabe, dass die Summe der vier Werte von n mindestens 1 beträgt.

Es sei hier klargestellt, dass sich die Werte der Variablen n jeweils auf ein individuelles Molekül der zuvor beschriebenen Verbindungen der Formeln I und la beziehen und damit zwangsläufig ganzzahlig sind. Betrachtet man hingegen ein Ensemble an Molekülen wie es synthesebedingt üblicherweise erhalten wird, so ergeben sich statistisch gesehen natürlich rationale Zahlenwerte für die mittlere Anzahl der an jedem Benzolring des Phthalocyaningrundgerüsts gebundenen Reste R.

Die Phthalocyanine der Formeln I und la können aus den unsubstituierten Phthalocyaninen durch Umsetzung mit den entsprechenden hydroxymethylierten Verbindungen in einer konzentrierten Säure erhalten werden (Methode A), wobei die hydroxymethylierten Verbindungen nach dem Fachmann allgemein bekannten Methoden zugänglich sind. Die Herstellung von Hydroxymethyllactamen aus den Lactamen wird beispielsweise in den Schriften US 4,769,454 und US 3,073,843 beschrieben.

Desweiteren können die Phthalocyanine der Formeln I und la aber auch mit stickstoffhaltigen Verbindungen wie Amiden, Lactamen, Imidazolen usw. und Paraformaldehyd in Gegenwart von Säure zu den gewünschten Markierungsstoffen umgesetzt werden (Methode B).

Durch die Reaktionsbedingungen (Temperatur, Reaktionszeit, Konzentration, Überschuß der Hydroxymethylverbindung etc.) wird der Substitutionsgrad des Phthalocyanins und damit dessen Löslichkeit gesteuert. Der Substitutionsgrads lässt sich beispielsweise massenspektroskopisch ermitteln.

### Phthalocyanin 1:

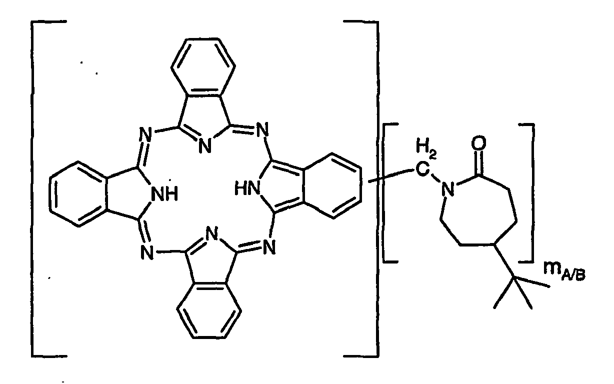

Die Verbindung wurde nach beiden Methoden A und B hergestellt:

### Methode A:

100 g Polyphosphorsäure wurden bei 40 bis 45 °C vorgelegt, 5,14 g (0,010 mol) metallfreies Phthalocyanin innerhalb von 20 Minuten eingetragen, die Mischung 30 Minuten nachgerührt und innerhalb von 20 Minuten 8,0 g (0,040 mol) 4-tert.-Butyl-N-hydroxymethylcaprolactam zugegeben. Es wurde 8 Stunden bei 105°C nachrühren und dann auf 50°C abkühlen gelassen, bevor mit 500 g Eiswasser verdünnt und anschließend abgesaugt wurde. Den feuchten Rückstand nahm man in 600 ml Wasser auf und stellte mit konzentrierter Natronlauge auf einen pH-Wert von 12,5 ein. Es wurde 2 Stunden nachgerührt, abfiltriert und im Vakuum getrocknet. Die Ausbeute betrug 7 g. Der mittlere Wert für *m*_{A} in der zuvor gezeigten Formel betrug 3 bis 4.

### Methode B:

100 g Polyphosphorsäure wurden bei 40 bis 45°C vorgelegt und 5,14 g (0,010 mol) metallfreies Phthalocyanin und 2,2 g (0,075 mol) Paraformaldehyd innerhalb von 20 Minuten eingetragen. Dann ließ man die Mischung 4 Stunden bei 50°C nachrühren und gab 12,7 g (0,075 mol) 4-tert.-Butylcaprolactam hinzu. Es wurde 7,5 Stunden bei 105°C nachrühren und dann auf 50°C abkühlen gelassen, bevor mit 500 g Eiswasser verdünnt und anschließend abgesaugt wurde. Den feuchten Rückstand nahm man in 600 ml Wasser auf und stellte mit konzentrierter Natronlauge auf einen pH-Wert von 12,5 ein. Es wurde 2 Stunden nachgerührt, abfiltriert und im Vakuum getrocknet. Die Ausbeute betrug 10 g. Der mittlere Wert für m_{B} in der zuvor gezeigten Formel betrug etwa 4.

In analoger Weise wurden die nachfolgend gezeigten Phthalocyanine 2 bis 5 hergestellt:

### Phthalocyanin 2 (hergestellt nach Methode A (m_{A} 3 bis 4) und B (m_{B} ca. 4)):

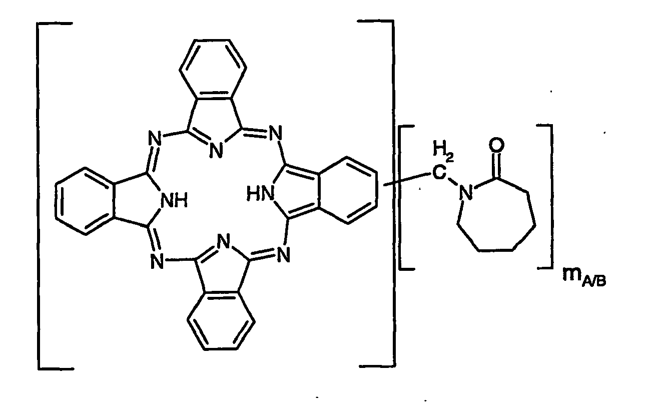

### Phthalocyanin 3 (hergestellt nach Methode B (m_{B} ca. 4)):

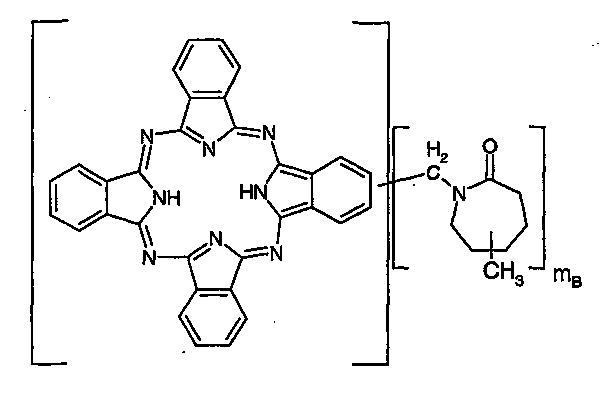

### Phthalocyanin 4 (hergestellt nach Methode B (m_{B} ca. 4)):

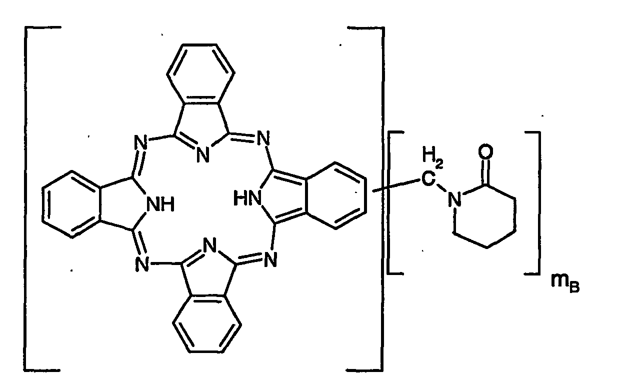

### Phthalocyanin 5 (hergestellt nach Methode B (m_{B} ca. 4)):

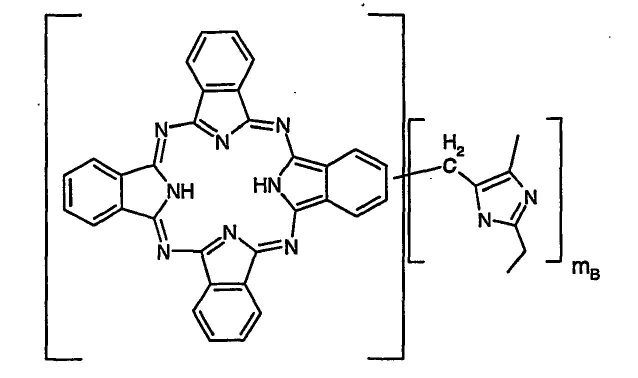

### Phthalocyanin 6 (Vergleich; hergestellt gemäß Beispiel 1 von WO 98/52950 A1):

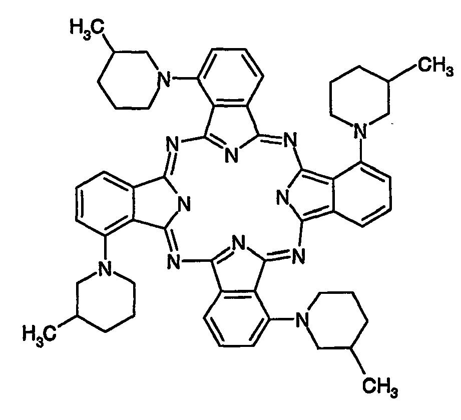

### Langzeitstabilität:

Die Langzeitstabilität der Phthalocyanine 1 und 2 und des Phthalocyanins 6 (Vergleich) wurde in Gegenwart eines handelsübliches Detergens für Mineralölkraftstoffe (Polyisobutenamin (PlBA); Lösung mit PIBA-Gehalt von 50 Gew.-%) untersucht. Hierzu löste man 50 bis 100 mg der jeweiligen Verbindung in 50 ml Shellsol AB oder, sofern die Löslichkeit der Verbindung in Shellsol AB nicht ausreichend war, diese erst mit ca. 5 ml Isopropanol oder N-Methylpyrrolidon an und füllte dann mit Shellsol AB auf 50 ml auf. Anschliessend wurde die Lösung durch einen Papier-Faltenfilter filtriert.

1 bis 3 ml des Filtrats wurden mit Detergens auf 10 ml ausgefüllt (entsprechend einer Konzentration der jeweiligen Verbindung von 0,01 bis 0,08 %) und in 1-mm-Küvetten gegen die entsprechende nicht-additivierte Referenz gemessen.

Die Proben wurden in 10-ml-Ampullen gefüllt, luftdicht verschlossen und bei 50°C im Wasserbad gelagert.

Die Ergebnisse sind in der nachfolgenden Tabellen wiedergegeben. Bei allen Messungen wurden auf die Anfangsextinktion normiert.

Vergleich der Langzeitstabilität bei 50°C gegenüber dem Detergens

| Phthalocyanin | Lagerzeit (h) | Absorptions-maximum (nm) | Extinktion (normiert) |
|---|---|---|---|
| 6 (Vergleich) | 0 | 768 | 1,00 |
| | 114 | | 0,33 |
| | 161 | | 0,20 |
| | 283 | | 0,05 |
| 1 | 0 | 599 | 1,00 |
| | 16 | | 0,98 |
| | 163 | | 0,97 |
| | 474 | | 0,95 |
| | 524 | | 0,93 |
| 2 | 0 | 695 | 1,00 |
| | 114 | | 0,89 |
| | 161 | | 0,88 |
| | 283 | | 0,78 |
| | 474 | | 0,77 |
| | 668 | | 0,81 |

Während im Falle des Phthalocyanins 6 (Vergleich) die normierte Extiktion nach 283 Stunden auf 5% des ursprünglichen Wertes abgesunken war, beliefen sich die Werte für die Phthalocyanine 1 und 2 nach deutlich längeren Lagerzeiten von 524 bzw. 668 Stunden immer noch auf 93 bzw. 81% der ursprünglichen Werte.

## Patentansprüche

1. Verwendung von Phthalocyaninen der Formel I 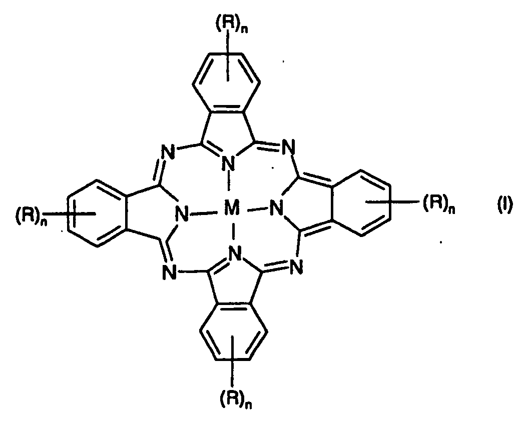 als Markierungsstoffe für Flüssigkeiten,
wobei in Formel I bedeuten
M zweimal Wasserstoff, zweimal Lithium, Magnesium, Zink, Kupfer, Nickel, VO, TiO, AlCl, AlOCOCH₃, AlOCOCF₃, SiCl₂ oder Si(OH)₂,
R gleiche oder voneinander verschiedene Gruppierungen ausgewählt aus der Gruppe bestehend aus -CH₂-N(-X¹-R¹)(-X²-R²) und -CH₂-Het,
X¹, X² unabhängig voneinander eine Carbonylgruppe oder eine chemische Ein- fachbindung,
R¹ C₁-C₂₀-Alkyl, das gegebenenfalls durch 1 bis 4 Sauerstoffatome in Ether- funktion unterbrochen ist,
C₅-C₇-Cycloalkyl, das gegebenenfalls mit einer oder mehreren C₁-C₂₀-Al- kylgruppen, die gegebenenfalls durch 1 bis 4 Sauerstoffatome in Ether- funktion unterbrochen sind, substituiert ist,
gesättigter heterocyclischer fünf- oder sechsgliedriger Rest, der gege- benenfalls mit einer oder mehreren C₁-C₂₀-Alkylgruppen, die gegebe- nenfalls durch 1 bis 4 Sauerstoffatome in Etherfunktion unterbrochen sind, substituiert ist,
C₆-C₁₀-Aryl, das gegebenenfalls mit einem oder mehreren Halogen, Cyano, Nitro, Hydroxy, Amino, C₁-C₂₀-Alkyl, das gegebenenfalls durch 1 bis 4 Sauerstoffatome in Etherfunktion unterbrochen ist, C₁-C₂₀-Alkoxy. C₁-C₂₀-Alkylamino oder C₁-C₂₀-Dialkylamino substituiert ist,
Heteroaryl mit 3 bis 12 Kohlenstoffatomen, das gegebenenfalls mit ei- nem oder mehreren C₁-C₂₀-Alkyl, das gegebenenfalls durch 1 bis 4 Sau- erstoffatome in Etherfunktion unterbrochen ist, C₁-C₂₀-Alk-oxy, C₁-C₂₀-Al- kylamino oder C₁-C₂₀-Dialkylamino substituiert ist,
C₆-C₁₀-Aryl-C₁-C₄-alkyl, das im Arylrest gegebenenfalls mit einem oder mehreren Halogen, Cyano, Nitro, Hydroxy, Amino, C₁-C₂₀-Alkyl, das ge- gebenenfalls durch 1 bis 4 Sauerstoffatome in Etherfunktion unterbro- chen ist, C₁-C₂₀-Alkoxy, C₁-C₂₀-Alkylamino oder C₁-C₂₀-Dialkylamino substituiert ist,
oder
Heteroaryl-C₁-C₄-alkyl mit 3 bis 12 Kohlenstoffatomen im Hetero-aryl- rest, wobei letzterer gegebenenfalls mit einem oder mehreren C₁-C₂₀-Al- kyl, das gegebenenfalls durch 1 bis 4 Sauerstoffatome in Etherfunktion unterbrochen ist, C₁-C₂₀-Alkoxy, C₁-C₂₀-Alkylamino oder C₁-C₂₀-Dialkyl- amino substituiert ist,
R² Wasserstoff oder unabhängig von R¹ die Bedeutung von R¹, wobei für den Fall, dass X² einer Carbonylgruppe entspricht, R² nicht die Bedeu- tung von Wasserstoff besitzt,
Het gesättigter heterocyclischer fünf-, sechs- oder siebengliedriger Rest, der gegebenenfalls mit einer oder mehreren C₁-C₂₀-Alkyl-gruppen, die ge- gebenenfalls durch 1 bis 4 Sauerstoffatome in Etherfunktion unterbro- chen sind, substituiert und in welchem gegebenenfalls eine CH₂-Gruppe durch eine Carbonylgruppe ersetzt ist,
oder
Heteroaryl mit 3 bis 12 Kohlenstoffatomen, das gegebenenfalls mit einer oder mehreren C₁-C₂₀-Alkylgruppen, die gegebenenfalls durch 1 bis 4 Sauerstoffatome in Etherfunktion unterbrochen sind, substituiert ist,
wobei die Anbindung des gesättigten heterocyclischen Restes oder des Heteroaryls an die CH₂-Gruppe der Gruppierung -CH₂-Het entweder über ein geeignetes Heteroatom oder ein Kohlenstoffatom erfolgt,
und
n jeweils unabhängig voneinander Werte von 0, 1, 2, 3 oder 4, mit der Maßgabe, dass die Summe der vier Werte von n mindestens 1 beträgt.

2. Verwendung nach Anspruch 1, **dadurch gekennzeichnet, dass** in Formel I bedeuten
M zweimal Wasserstoff, zweimal Lithium, Magnesium, Zink, Kupfer, Nickel, VO, TiO, AlCl, AlOCOCH₃, AlOCOCF₃, SiCl₂ oder Si(OH)₂,
R gleiche oder voneinander verschiedene Gruppierungen -CH₂-Het,
Het gesättigter heterocyclischer fünf-, sechs- oder siebengliedriger Rest, der gegebenenfalls mit einer oder mehreren C₁-C₁₅-Alkyl-gruppen, die ge- gebenenfalls durch 1 bis 4 Sauerstoffatome in Etherfunktion unterbro- chen sind, substituiert und in welchem gegebenenfalls eine CH₂-Gruppe durch eine Carbonylgruppe ersetzt ist,
oder
Heteroaryl mit 3 bis 5 Kohlenstoffatomen, das gegebenenfalls mit einer oder mehreren C₁-C₁₅-Alkylgruppen, die gegebenenfalls durch 1 bis 4 Sauerstoffatome in Etherfunktion unterbrochen sind, substituiert ist,
wobei die Anbindung des gesättigten heterocyclischen Restes oder des Heteroaryls an die CH₂-Gruppe der Gruppierung -CH₂-Het entweder über ein geeignetes Heteroatom oder ein Kohlenstoffatom erfolgt,
und
n jeweils unabhängig voneinander Werte von 0, 1 oder 2, mit der Maßga- be, dass die Summe der vier Werte von n mindestens 1 beträgt.

3. Verwendung nach Anspruch 1, **dadurch gekennzeichnet, dass** in Formel I bedeuten
M zweimal Wasserstoff, zweimal Lithium, Magnesium, Zink, Kupfer, Nickel, VO, TiO, AlCl, AlOCOCH₃, AlOCOCF₃, SiCl₂ oder Si(OH)₂,
R gleiche oder voneinander verschiedene Gruppierungen -CH₂-Het,
Het gesättigter heterocyclischer fünf-, sechs- oder siebengliedriger Rest, der gegebenenfalls mit einer oder mehreren C₁-C₄-Alkyl-gruppen substituiert und in welchem gegebenenfalls eine CH₂-Gruppe durch eine Carbonyl- gruppe ersetzt ist,
oder
Heteroaryl mit 3 bis 5 Kohlenstoffatomen, das gegebenenfalls mit einer oder mehreren C₁-C₄-Alkylgruppen substituiert ist,
wobei die Anbindung des gesättigten heterocyclischen Restes oder des Heteroaryls an die CH₂-Gruppe der Gruppierung -CH₂-Het entweder über ein geeignetes Heteroatom oder ein Kohlenstoffatom erfolgt,
und
n jeweils unabhängig voneinander Werte von 0, 1 oder 2, mit der Maßga- be, dass die Summe der vier Werte von n mindestens 1 beträgt.

4. Verwendung nach Anspruch 1, **dadurch gekennzeichnet, dass** in Formel I bedeuten
M zweimal Wasserstoff,
R gleiche oder voneinander verschiedene Gruppierungen -CH₂-Het,
Het gesättigter stickstoffhaltiger fünf-, sechs- oder siebengliedriger Rest, der gegebenenfalls mit einer oder mehreren C₁-C₄-Alkyl-gruppen substituiert und in welchem die dem Stickstoffatom benachbarte CH₂-Gruppe durch eine Carbonylgruppe ersetzt ist,
oder
stickstoffhaltiges Heteroaryl mit 3 bis 5 Kohlenstoffatomen, das gegebe- nenfalls mit einer oder mehreren C₁-C₄-Alkylgruppen substituiert ist,
wobei die Anbindung des gesättigten heterocyclischen Restes oder des Heteroaryls an die CH₂-Gruppe der Gruppierung -CH₂-Het entweder über ein geeignetes Stickstoffatom oder ein Kohlenstoffatom erfolgt,
und
n jeweils unabhängig voneinander Werte von 0, 1 oder 2, mit der Maßga- be, dass die Summe der vier Werte von n mindestens 1 beträgt.

5. Verwendung nach Anspruch 1, **dadurch gekennzeichnet, dass** in Formel I bedeuten
M zweimal Wasserstoff,
R gleiche Gruppierungen -CH₂-Het,
Het ein γ-Butyrolactam-, δ-Valerolactam- oder ε-Caprolactamrest, der gege- benenfalls mit einer oder mehreren C₁-C₄-Alkylgruppen substituiert und an die CH₂-Gruppe der Gruppierung -CH₂-Het über das Stickstoffatom gebunden ist,
und
n jeweils unabhängig voneinander Werte von 0, 1 oder 2, mit der Maßga- be, dass die Summe der vier Werte von n mindestens 1 beträgt.

6. Verwendung nach Anspruch 1, **dadurch gekennzeichnet, dass** in Formel I bedeuten
M zweimal Wasserstoff, zweimal Lithium, Magnesium, Zink, Kupfer, Nickel, VO, TiO, AlCl, AlOCOCH₃, AlOCOCF₃, SiCl₂ oder Si(OH)₂,
R gleiche oder voneinander verschiedene Gruppierungen -CH₂-N(-X¹-R¹)(- X²-R²),
X¹, X² unabhängig voneinander eine Carboxylgruppe oder eine chemische Ein- fachbindung,
R¹ C₁-C₁₅-Alkyl, das gegebenenfalls durch 1 bis 4 Sauerstoffatome in Ether- funktion unterbrochen ist,
Cyclohexyl, das gegebenenfalls mit einer oder mehreren C₁-C₁₅-Alkyl- gruppen, die gegebenenfalls durch 1 bis 4 Sauerstoffatome in Etherfunk- tion unterbrochen sind, substituiert ist,
gesättigter heterocyclischer fünf- oder sechsgliedriger Rest, der gege- benenfalls mit einer oder mehreren C₁-C₁₅-Alkylgruppen, die gegebe- nenfalls durch 1 bis 4 Sauerstoffatome in Etherfunktion unterbrochen sind, substituiert ist,
C₆-C₁₀-Aryl, das gegebenenfalls mit einem oder mehreren C₁-C₁₅-Alkyl, das gegebenenfalls durch 1 bis 4 Sauerstoffatome in Etherfunktion un- terbrochen ist, C₁-C₁₅-Alkoxy, C₁-C₁₆-Alkylamino oder C₁-C₁₅-Dialkyl- amino substituiert ist,
Heteroaryl mit 3 bis 5 Kohlenstoffatomen, das gegebenenfalls mit einem oder mehreren C₁-C₁₅-Alkyl, das gegebenenfalls durch 1 bis 4 Sauer- stoffatome in Etherfunktion unterbrochen ist, C₁-C₁₅-Alk-oxy, C₁-C₁₅-Al- kylamino oder C₁-C₁₀-Dialkylamino substituiert ist,
Phenyl-C₁-C₄-alkyl, das im Arylrest gegebenenfalls mit einem oder meh- reren C₁-C₁₅-Alkyl, das gegebenenfalls durch 1 bis 4 Sauerstoffatome in Etherfunktion unterbrochen ist, C₁-C₁₅-Alkoxy, C₁-C₁₅-Alkylamino oder C₁-C₁₅-Dialkylamino substituiert ist,
oder
Heteroaryl-C₁-C₄-alkyl mit 3 bis 5 Kohlenstoffatomen im Hetero-arylrest, wobei letzterer gegebenenfalls mit einem oder mehreren C₁-C₁₅-Alkyl, das gegebenenfalls durch 1 bis 4 Sauerstoffatome in Etherfunktion un- terbrochen ist, C₁-C₁₅-Alkoxy, C₁-C₁₅-Alkylamino oder C₁-C₁₅-Dialkyl- amino substituiert ist,
R² Wasserstoff oder unabhängig von R¹ die Bedeutung von R¹, wobei für den Fall, dass X² einer Carbonylgruppe entspricht, R² nicht die Bedeu- tung von Wasserstoff besitzt,
und
n jeweils unabhängig voneinander Werte von 0, 1 oder 2, mit der Maßga- be, dass die Summe der vier Werte von n mindestens 1 beträgt.

7. Verwendung nach Anspruch 1, **dadurch gekennzeichnet, dass** in Formel I bedeuten
M zweimal Wasserstoff,
R gleiche oder voneinander verschiedene Gruppierungen -CH₂-N(-X¹-R¹)(-X²-R²,
X¹, X² unabhängig voneinander eine Carbonylgruppe oder eine chemische Ein- fachbindung,
R¹ C₁-C₁₅-Alkyl, das gegebenenfalls durch 1 bis 4 Sauerstoffatome in Ether- funktion unterbrochen ist,
Cyclohexyl, das gegebenenfalls mit einer oder mehreren C₁-C₁₅-Alkyl- gruppen, die gegebenenfalls durch 1 bis 4 Sauerstoffatome in Etherfunk- tion unterbrochen sind, substituiert ist,
gesättigter heterocyclischer fünf- oder sechsgliedriger Rest, der gege- benenfalls mit einer oder mehreren C₁-C₁₅-Alkylgruppen, die gege- benenfalls durch 1 bis 4 Sauerstoffatome in Etherfunktion unterbrochen sind, substituiert ist,
Phenyl, das gegebenenfalls mit einem oder mehreren C₁-C₁₅Alkyl, das gegebenenfalls durch 1 bis 4 Sauerstoffatome in Etherfunktion unterbro- chen ist, C₁-C₁₅-Alkoxy, C₁-C₁₅-Alkylamino oder C₁-C₁₅-Dialkylamino substituiert ist,
oder
Phenyl-C₁-C₄-alkyl, das im Arylrest gegebenenfalls mit einem oder meh- reren C₁-C₁₅-Alkyl, das gegebenenfalls durch 1 bis 4 Sauerstoffatome in Etherfunktion unterbrochen ist, C₁-C₁₅-Alkoxy, C₁-C₁₅-Alkylamino oder C₁-C₁₅-Dialkylamino substituiert ist,
R² Wasserstoff oder unabhängig von R¹ die Bedeutung von R¹, wobei für den Fall, dass X² einer Carbonylgruppe entspricht, R² nicht die Bedeu- tung von Wasserstoff besitzt,
und
n jeweils unabhängig voneinander Werte von 0, 1 oder 2, mit der Maßga- be, dass die Summe der vier Werte von n mindestens 1 beträgt.

8. Verwendung von Phthalocyaninen der Formel I gemäß einem oder mehreren der Ansprüche 1 bis 7 als Markierungsstoffe für Mineralöle.

9. Flüssigkeiten enthaltend mindestens ein Phthalocyanin der Formel I gemäß einem oder mehreren der Ansprüche 1 bis 7 als Markierungsstoff.

10. Mineralöle enthaltend mindestens ein Phthalocyanin der Formel I gemäß einem oder mehreren der Ansprüche 1 bis 7 als Markierungsstoff.

11. Phthalocyanine der Formel la 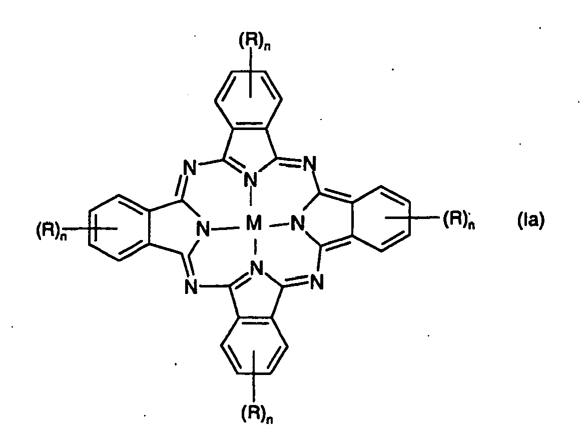 wobei in Formel la bedeuten
M zweimal Wasserstoff,
R gleiche oder voneinander verschiedene Gruppierungen -CH₂-Het,
Het gesättigter stickstoffhaltiger fünf-, sechs- oder siebengliedriger Rest, der gegebenenfalls mit einer oder mehreren C₁-C₄-Alkl-gruppen substituiert und in welchem die dem Stickstoffatom benachbarte CH₂-Gruppe durch eine Carbonylgruppe ersetzt ist,
wobei die Anbindung des gesättigten heterocyclischen Restes an die CH₂-Gruppe der Gruppierung -CH₂-Het entweder über ein geeignetes Stickstoffatom oder ein Kohlenstoffatom erfolgt,
und
n jeweils unabhängig voneinander Werte von 0, 1 oder 2, mit der Maßga- be, dass die Summe der vier Werte von n mindestens 1 beträgt.

12. Phthalocyanine nach Anspruch 11 wobei in Formel la bedeuten
M zweimal Wasserstoff,
R gleiche Gruppierungen -CH₂-Het,
Het ein γ-Butyrolactam-, ö-Valerolactam- oder ε-Caprolactamrest, der gege- benenfalls mit einer oder mehreren C₁-C₄-Alkylgruppen substituiert und an die CH₂-Gruppe der Gruppierung -CH₂Het über das Stickstoffatom gebunden ist,
und
n jeweils unabhängig voneinander Werte von 0, 1 oder 2, mit der Maßga- be, dass die Summe der vier Werte von n mindestens 1 beträgt.

## Claims

1. The use of phthalocyanines of the formula I 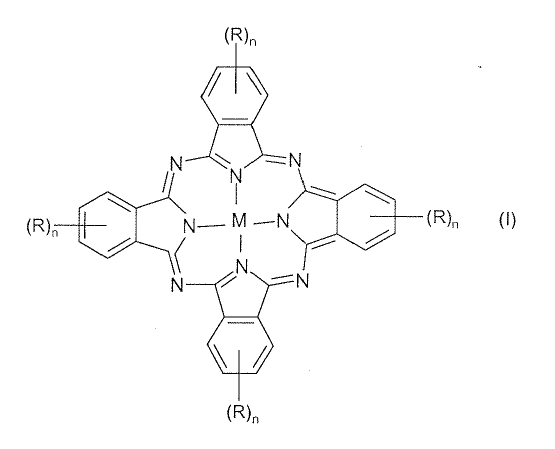 as markers for liquids,
where, in formula I,
M is twice hydrogen, twice lithium, magnesium, zinc, copper, nickel, VO, TiO, AlCl, AlOCOCH₃, AlOCOCF₃, SiCl₂ or Si(OH)₂,
R are each identical or different moieties selected from the group consisting of -CH₂-N(-X¹-R¹)(-X²-R²) and -CH₂-Het,
X¹, X² are each independently a carbonyl group or a chemical single bond,
R¹ is C₁-C₂₀-alkyl which is optionally interrupted by from 1 to 4 oxygen atoms in ether function,
C₅-C₇-cycloalkyl which is optionally substituted by one or more C₁- C₂₀-alkyl groups which are optionally interrupted by from 1 to 4 oxygen atoms in ether function,
saturated heterocyclic five- or six-membered radical which is optionally substituted by one or more C₁-C₂₀-alkyl groups which are optionally interrupted by from 1 to 4 oxygen atoms in ether function,
C₆-C₁₀-aryl which is optionally substituted by one or more halogen, cyano, nitro, hydroxyl, amino, C₁-C₂₀-alkyl which is optionally substituted by from 1 to 4 oxygen atoms in ether function, C₁-C₂₀- alkoxy, C₁-C₂₀-alkylamino or C₁-C₂₀-dialkylamino,
heteroaryl having from 3 to 12 carbon atoms which is optionally substituted by one or more C₁-C₂₀-alkyl which is optionally interrupted by from 1 to 4 oxygen atoms in ether function, C₁-C₂₀- alkoxy, C₁-C₂₀-alkylamino or C₁-C₂₀-dialkylamino,
C₆-C₁₀-aryl-C₁-C₄-alkyl which is optionally substituted in the aryl radical by one or more halogen, cyano, nitro, hydroxyl, amino, C₁- C₂₀-alkyl which is optionally interrupted by from 1 to 4 oxygen atoms in ether function, C₁-C₂₀-alkoxy, C₁-C₂₀-alkylamino or C₁-C₂₀- dialkylamino,
or
heteroaryl-C₁-C₄-alkyl having from 3 to 12 carbon atoms in the heteroaryl radical, the latter optionally being substituted by one or more C₁-C₂₀-alkyl which is optionally interrupted by from 1 to 4 oxygen atoms in ether function, C₁-C₂₀-alkoxy, C₁-C₂₀-alkylamino or C₁-C₂₀-dialkylamino,
R² is hydrogen or, independently of R¹, as defined for R¹, and, in the case that X² is a carbonyl group, R² is not defined as hydrogen,
Het is a saturated heterocyclic five-, six- or seven-membered radical which is optionally interrupted by one or more C₁-C₂₀-alkyl groups which is optionally interrupted by from 1 to 4 oxygen atoms in ether function, and in which one CH₂ group is optionally replaced by a carbonyl group,
or
heteroaryl having from 3 to 12 carbon atoms which is optionally substituted by one or more C₁-C₂₀-alkyl groups which are optionally interrupted by from 1 to 4 oxygen atoms in ether function,
the saturated heterocyclic radical or the heteroaryl being bonded to the CH₂ group of the -CH₂-Het moiety either via a suitable heteroatom or a carbon atom,
and
n is in each case independently a value of 0, 1, 2, 3 or 4, with the proviso that the sum of the four values of n is at least 1.

2. The use according to claim 1, wherein, in formula I,
M is twice hydrogen, twice lithium, magnesium, zinc, copper, nickel, VO, TiO, AlCl, AlOCOCH₃, AlOCOCF₃, SiCl₂ or Si(OH)₂,
R are each identical or different -CH₂-Het moieties,
Het is a saturated heterocyclic five-, six- or seven-membered ring which is optionally substituted by one or more C₁-C₁₅-alkyl groups which are optionally interrupted by from 1 to 4 oxygen atoms in ether function, and in which one CH₂ group is optionally replaced by a carbonyl group,
or
heteroaryl having from 3 to 5 carbon atoms which is optionally substituted by one or more C₁-C₁₅-alkyl groups which are optionally interrupted by from 1 to 4 oxygen atoms in ether function,
the saturated heterocyclic radical or the heteroaryl being bonded to the CH₂ group of the -CH₂-Het moiety either via a suitable heteroatom or a carbon atom,
and
n in each case independently has a value of 0, 1 or 2, with the proviso that the sum of the four values of n is at least 1.

3. The use according to claim 1, wherein, in formula I,
M is twice hydrogen, twice lithium, magnesium, zinc, copper, nickel, VO, TiO, AlCl, AlOCOCH₃, AlOCOCF₃, SiCl₂ or Si(OH)₂,
R are identical or different -CH₂-Het moieties,
Het is a saturated heterocyclic five-, six- or seven-membered radical which is optionally substituted by one or more C₁-C₄-alkyl groups and in which one CH₂ group is optionally replaced by a carbonyl group,
or
heteroaryl having from 3 to 5 carbon atoms which is optionally substituted by one or more C₁-C₄-alkyl groups,
the saturated heterocyclic radical or the heteroaryl being bonded to the CH₂ group of the -CH₂-Het moiety either via a suitable heteroatom or a carbon atom,
and
n is in each case independently a value of 0, 1 or 2, with the proviso that the sum of the four values of n is at least 1.

4. The use according to claim 1, wherein, in formula I**,**
M is twice hydrogen,
R are identical or different -CH₂-Het moieties,
Het is a saturated, nitrogen-containing, five-, six- or seven-membered radical which is optionally substituted by one or more C₁-C₄-alkyl groups and in which the CH₂ group adjacent to the nitrogen atom is replaced by a carbonyl group,
or
nitrogen-containing heteroaryl having from 3 to 5 carbon atoms which is optionally substituted by one or more C₁-C₄-alkyl groups,
the saturated heterocyclic radical or the heteroaryl being bonded to the CH₂ group of the -CH₂-Het moiety either via a suitable nitrogen atom or a carbon atom,
and
n is in each case independently a value of 0, 1 or 2, with the proviso that the sum of the four values of n is at least 1.

5. The use according to claim 1, wherein, in formula I,
M is twice hydrogen,
R are identical -CH₂-Het moieties,
Het is a γ-butyrolactam, δ-valerolactam or ε-caprolactam radical which is optionally substituted by one or more C₁-C₄-alkyl groups and is bonded to the CH₂ group of the -CH₂-Het moiety via the nitrogen atom,
and
n is in each case independently a value of 0, 1 or 2, with the proviso that the sum of the four values of n is at least 1.

6. The use according to claim 1, wherein, in formula I,
M is twice hydrogen, twice lithium, magnesium, zinc, copper, nickel, VO, TiO, AlCl, AlOCOCH₃, AlOCOCF₃, SiCl₂ or Si(OH)₂,
R are each identical or different moieties selected from the group consisting of -CH₂-N(-X¹-R¹)(-X²-R²),
X¹, X² are each independently a carbonyl group or a chemical single bond,
R¹ is C₁-C₁₅-alkyl which is optionally interrupted by from 1 to 4 oxygen atoms in ether function,
cyclohexyl which is optionally substituted by one or more C₁-C₁₅- alkyl groups which are optionally interrupted by from 1 to 4 oxygen atoms in ether function,
saturated heterocyclic five- or six-membered radical which is optionally substituted by one or more C₁-C₁₅-alkyl groups which are optionally interrupted by from 1 to 4 oxygen atoms in ether function,
C₆-C₁₀-aryl which is optionally substituted by one or more C₁-C₁₅- alkyl which is optionally substituted by from 1 to 4 oxygen atoms in ether function, C₁-C₁₅-alkoxy, C₁-C₁₅-alkylamino or C₁-C₁₅- dialkylamino,
heteroaryl having from 3 to 5 carbon atoms which is optionally substituted by one or more C₁-C₁₅-alkyl which is optionally interrupted by from 1 to 4 oxygen atoms in ether function, C₁-C₁₅- alkoxy, C₁-C₁₅-alkylamino or C₁-C₁₅-dialkylamino,
phenyl-C₁-C₄-alkyl which is optionally substituted in the aryl radical by one or more C₁-C₁₅-alkyl which is optionally interrupted by from 1 to 4 oxygen atoms in ether function, C₁-C₅-alkoxy, C₁-C₁₅- alkylamino or C₁-C₁₅-dialkylamino,
or
heteroaryl-C₁-C₄-alkyl having from 3 to 5 carbon atoms in the heteroaryl radical, the latter optionally being substituted by one or more C₁-C₁₅-alkyl which is optionally interrupted by from 1 to 4 oxygen atoms in ether function, C₁-C₁₅-alkoxy, C₁-C₁₅-alkylamino or C₁-C₁₅-dialkylamino,
R² is hydrogen or, independently of R¹, as defined for R¹, and, in the case that X² is a carbonyl group, R² is not defined as hydrogen,
and
n is in each case independently a value of 0, 1 or 2, with the proviso that the sum of the four values of n is at least 1.

7. The use according to claim 1, wherein, in formula I,
M is twice hydrogen,
R are each identical or different moieties selected from the group consisting of -CH₂-N(-X¹-R¹)(-X²-R²),
X¹, X² are each independently a carbonyl group or a chemical single bond,
R¹ is C₁-C₁₅-alkyl which is optionally interrupted by from 1 to 4 oxygen atoms in ether function, cyclohexyl which is optionally substituted by one or more C₁-C₁₅ alkyl groups which are optionally interrupted by from 1 to 4 oxygen atoms in ether function,
saturated heterocyclic five- or six-membered radical which is optionally substituted by one or more C₁-C₁₅-alkyl groups which are optionally interrupted by from 1 to 4 oxygen atoms in ether function,
phenyl which is optionally substituted by one or more C₁-C₁₅-alkyl which is optionally substituted by from 1 to 4 oxygen atoms in ether function, C₁-C₁₅-alkoxy, C₁-C₁₅-alkylamino or C₁-C₁₅-dialkylamino,
or
phenyl-C₁-C₄-alkyl which is optionally substituted in the aryl radical by one or more C₁-C₁₅-alkyl which is optionally interrupted by from 1 to 4 oxygen atoms in ether function, C₁-C₁₅-alkoxy, C₁-C₁₅- alkylamino or C₁-C₁₅-dialkylamino,
R² is hydrogen or, independently of R¹, as defined for R¹ and, in the case that X² is a carbonyl group, R² is not defined as hydrogen,
and
n is in each case independently a value of 0, 1 or 2, with the proviso that the sum of the four values of n is at least 1.

8. The use of phthalocyanines of the formula I according to one or more of claims 1 to 7 as markers for mineral oils.

9. A liquid comprising at least one phthalocyanine of the formula I according to one or more of claims 1 to 7 as a marker.

10. A mineral oil comprising at least one phthalocyanine of the formula I according to one or more of claims 1 to 7 as a marker.

11. A phthalocyanine of the formula la 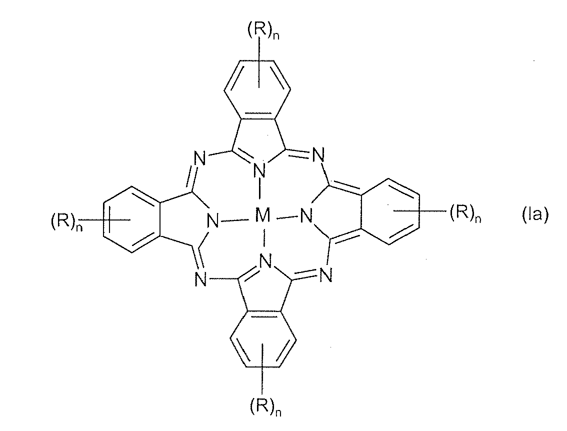 where, in formula Ia,
M is twice hydrogen,
R are identical or different -CH₂-Het moieties,
Het is a saturated heterocyclic five-, six- or seven-membered radical which is optionally substituted by one or more C₁-C₄-alkyl groups and in which the CH₂ group adjacent to the nitrogen atom is optionally replaced by a carbonyl group,
the saturated heterocyclic radical being bonded to the CH₂ group of the -CH₂-Het moiety either via a suitable heteroatom or a carbon atom,
and
n is in each case independently a value of 0, 1 or 2, with the proviso that the sum of the four values of n is at least 1.

12. A phthalocyanine according to claim 11, where, in formula Ia,
M is twice hydrogen,
R are identical -CH₂-Het moieties,
Het is a γ-butyrolactam, δ-valerolactam or ε-caprolactam radical which is optionally substituted by one or more C₁-C₄-alkyl groups and is bonded to the CH₂ group of the -CH₂-Het moiety via nitrogen atom,
and
n is in each case independently a value of 0, 1 or 2, with the proviso that the sum of the four values of n is at least 1.

## Revendications

1. Utilisation de phtalocyanines de la formule I 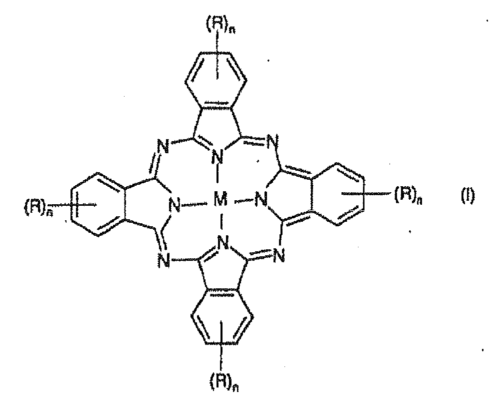 en tant que substances de marquage pour fluides, étant donné que, dans la formule I,
M désigne deux fois l'hydrogène, deux fois le lithium, le magnésium, le zinc, le cuivre, le nickel, VO, TiO, AlCl, AlOCOCH₃, AlOCOCF₃, SiCl₂ ou Si(OH)₂,
R désigne des groupements identiques ou différents les uns des autres sélectionnés parmi le groupe se composant de -CH₂-N(-X¹-R¹)(-X²-R²) et -CH₂-Het,
X¹, X² désignent, indépendamment l'un de l'autre, un groupement carbonyle ou une liaison chimique simple,
R¹ désigne un radical C₁-C₂₀-alkyle, qui est interrompu, le cas échéant, par de 1 à 4 atomes d'oxygène en formant une fonction éther,
un radical C₅-C₇-cycloalkyle, qui est substitué, le cas échéant, à l'aide d'un ou de plusieurs groupements C₁-C₂₀-alkyles, qui sont, le cas échéant, interrompus par de 1 à 4 atomes d'oxygène en formant une fonction éther,
un radical hétérocyclique saturé à cinq ou six membres, qui est substitué, le cas échéant, à l'aide d'un ou de plusieurs groupements C₁-C₂₀-alkyles, qui sont, le cas échéant, interrompus par de 1 à 4 atomes d'oxygène en formant une fonction éther,
un radical C₆-C₁₀-aryle, qui est substitué, le cas échéant, à l'aide d'un ou de plusieurs halogènes, un radical cyano, nitro, hydroxy, amino, C₁-C₂₀-alkyle, qui est interrompu, le cas échéant, par de 1 à 4 atomes d'oxygène en formant une fonction éther, un radical C₁-C₂₀-alkoxy, C₁-C₂₀-alkylamino ou C₁-C₂₀-dialkylamino,
un radical hétéroaryle ayant de 3 à 12 atomes de carbone, qui est substitué, le cas échéant, par un ou plusieurs radicaux C₁-C₂₀-alkyles, qui peuvent être interrompus, le cas échéant, par de 1 à 4 atomes d'oxygène en formant une fonction éther, un radical C₁-C₂₀-alkoxy, C₁-C₂₀-alkylamino ou C₁-C₂₀-dialkylamino,
un radical C₈-C₁₀-aryle-C₁-C₄-alkyle, qui est substitué, dans le radical aryle, le cas échéant, à l'aide d'un ou de plusieurs halogènes, un radical cyano, nitro, hydroxy, amino, C₁-C₂₀-alkyle, qui est interrompu, le cas échéant, par de 1 à 4 atomes d'oxygène en formant une fonction éther, un radical C₁-C₂₀-alkoxy, C₁-C₂₀-alkylamino ou C₁-C₂₀-dialkylamino,
ou
un radical hétéroaryle-C₁-C₄-alkyle ayant de 3 à 12 atomes de carbone dans le radical hétéroaryle, ce dernier étant substitué, le cas échéant, à l'aide d'un ou de plusieurs radicaux C₁-C₂₀-alkyle, qui est interrompu, le cas échéant, par de 1 à 4 atomes d'oxygène en formant une fonction éther, un radical C₁-C₂₀-alkoxy, C₁-C₂₀-alkylamino ou C₁-C₂₀-dialkylamino,
R² désigne l'hydrogène ou indépendamment de R¹, a la signification de R¹, étant donné qu'au cas où X² correspond à un groupement carbonyle, R² ne possède pas la signification d'hydrogène,
Het signifie un radical hétérocyclique saturé, à cinq, six ou sept membres, qui est substitué, le cas échéant, à l'aide d'un ou de plusieurs groupements C₁-C₂₀-alkyles, qui sont interrompus, le cas échéant, par de 1 à 4 atomes d'oxygène en formant une fonction éther, et dans lequel, le cas échéant, un groupement CH₂ est remplacé par un groupement carbonyle,
ou
un radical hétéroaryle ayant de 3 à 12 atomes de carbone, qui est substitué, le cas échéant, à l'aide d'un ou de plusieurs groupements C₁-C₂₀-alkyles, qui sont, le cas échéant, interrompus par de 1 à 4 atomes d'oxygène en formant une fonction éther,
la liaison du radical hétérocyclique saturé ou du radical hétéroaryle au groupement CH₂ du groupement -CH₂-Het se faisant ou par un hétéroatome approprié ou par un atome de carbone,
et
n désigne, indépendamment les unes des autres, des valeurs de 0, 1, 2, 3 ou 4, sous réserve que la somme des quatre valeurs de n soit d'au moins 1.

2. Utilisation selon la revendication 1, **caractérisée en ce que**, dans la formule I,
M désigne deux fois l'hydrogène, deux fois le lithium, le magnésium, le zinc, le cuivre, le nickel, VO, TiO, AlCl, AlOCOCH₃, AlOCOCF₃, SiCl₂ ou Si (OH)₂,
R désigne des groupements -CH₂-Het identiques ou différents les uns des autres,
Het signifie un radical hétérocyclique saturé à cinq, six ou sept membres, qui est substitué, le cas échéant, à l'aide d'un ou de plusieurs groupements C₁-C₁₅-alkyles, qui sont interrompus, le cas échéant, par de 1 à 4 atomes d'oxygène en formant une fonction éther, et dans lequel, le cas échéant, un groupement CH₂ est remplacé par un groupement carbonyle,
ou
un radical hétéroaryle ayant de 3 5 atomes de carbone, qui est substitué, le cas échéant, à l'aide d'un ou de plusieurs groupements C₁-C₁₅-alkyles, qui sont, le cas échéant, interrompus par de 1 à 4 atomes d'oxygène en formant une fonction éther,
la liaison du radical hétérocyclique saturé ou du radical hétéroaryle au groupement CH₂ du groupement -CH₂-Het se faisant ou par un hétéroatome approprié ou par un atome de carbone,
et
n désigne, indépendamment les unes des autres, des valeurs de 0, 1, ou 2, sous réserve que la somme des quatre valeurs de n soit d'au moins 1.

3. Utilisation selon la revendication 1, **caractérisé en ce que**, dans la formule I,
M désigne deux fois l'hydrogène, deux fois le lithium, le magnésium, le zinc, le cuivre, le nickel, VO, TiO, AlCl, AlOCOCH₃, AlOCOCF₃, SiCl₂ ou Si(OH)₂,
R désigne des groupements -CH₂-Het identiques ou différents les uns des autres,
Het signifie un radical hétérocyclique saturé, à cinq, six ou sept membres, qui est substitué, le cas échéant, à l'aide d'un ou de plusieurs groupements C₁-C₄-alkyles, et dans lequel, le cas échéant, un groupement CH₂ est remplacé par un groupement carbonyle,
ou
un radical hétéroaryle ayant de 3 à 5 atomes de carbone, qui est substitué, le cas échéant, à l'aide d'un ou de plusieurs groupements C₁-C₄-alkyles,
la liaison du radical hétérocyclique saturé ou du radical hétéroaryle au groupement CH₂ du groupement -CH₂-Het se faisant ou par un hétéroatome approprié ou par un atome de carbone,
et
n désigne, indépendamment les unes des autres, des valeurs de 0, 1, ou 2, sous réserve que la somme des quatre valeurs de n soit d'au moins 1.

4. Utilisation selon la revendication 1, **caractérisée en ce que**, dans la formule I,
M désigne deux fois l'hydrogène,
R désigne des groupements -CH₂-Het, identiques ou différents les uns des autres,
Het signifie un radical saturé, contenant de l'azote, à cinq, six ou sept membres, qui est substitué, le cas échéant, à l'aide d'un ou de plusieurs groupements C₁-C₄-alkyles, et dans lequel, le cas échéant, un groupement CH₂ est remplacé par un groupement carbonyle,
ou
un radical hétéroaryle, contenant de l'azote, ayant de 3 à 5 atomes de carbone, qui est substitué, le cas échéant, à l'aide d'un ou de plusieurs groupements C₁-C₄-alkyles,
la liaison du radical hétérocyclique saturé ou du radical hétéroaryle au groupement CH₂ du groupement -CH₂-Het se faisant ou par un hétéroatome approprié ou par un atome de carbone,
et
n désigne, indépendamment les unes des autres, des valeurs de 0, 1, ou 2, sous réserve que la somme des quatre valeurs de n soit d'au moins 1.

5. Utilisation selon la revendication 1, **caractérisée en ce que**, dans la formule I,
M désigne deux fois l'hydrogène,
R désigne des groupements -CH₂-Het identiques,
Het désigne un radical γ-butyrolactame, δ-valérolactame ou ε-caprolactame, qui est substitué, le cas échéant, à l'aide d'un ou de plusieurs groupements C₁-C₄-alkyles et auquel le groupement CH₂ du groupement CH₂-Het est lié à l'aide de l'atome d'azote,
et
n désigne, indépendamment les unes des autres, des valeurs de 0, 1, ou 2, sous réserve que la somme des quatre valeurs de n soit d'au moins 1.

6. Utilisation selon la revendication 1, **caractérisée en ce que**, dans la formule I,
M désigne deux fois l'hydrogène, deux fois le lithium, le magnésium, le zinc, le cuivre, le nickel, VO, TiO, AlCl, AlOCOCH₃, AlOCOCF₃, SiCl₂ ou Si(OH)₂,
R désigne des groupements -CH₂-N(-X¹-R¹) (-X²-R²) identiques ou différents les uns des autres,
X¹, X² désignent, indépendamment l'un de l'autre, un groupement carbonyle ou une liaison chimique simple,
R¹ désigne un radical C₁-C₁₅,-alkyle, qui est interrompu, le cas échéant, par de 1 à 4 atomes d'oxygène en formant une fonction éther,
un radical cyclohexyle, qui est substitué, le cas échéant, à l'aide d'un ou de plusieurs groupements C₁-C₁₅-alkyles, qui sont, le cas échéant, interrompus par de 1 à 4 atomes d'oxygène en formant une fonction éther,
un radical hétérocyclique saturé, à cinq ou six membres, qui est substitué, le cas échéant, à l'aide d'un ou de plusieurs groupements C₁-C₁₅-alkyles, qui sont, le cas échéant, interrompus par de 1 à 4 atomes d'oxygène en formant une fonction éther,
un radical C₆-C₁₀-aryle, qui est substitué, le cas échéant, à l'aide d'un ou de plusieurs radicaux C₁-C₁₅-alkyles, qui est interrompu, le cas échéant, par de 1 à 4 atomes d'oxygène en formant une fonction éther, un radical C₁-C₁₅-alkoxy, C₁-C₁₅-alkylamino ou C₁-C₁₅-dialkylamino,
un radical hétéroaryle ayant de 3 à 5 atomes de carbone, qui est substitué, le cas échéant, par un ou plusieurs radicaux C₁-C₁₅-alkyles, qui est interrompu, le cas échéant, par de 1 à 4 atomes d'oxygène en formant une fonction éther, un radical C₁-C₁₅-alkoxy, C₁-C₁₅-alkylamino ou C₁-C₁₅-dialkylamino,
un radical phényl-C₁-C₄-alkyle, qui est substitué, dans le radical aryle, le cas échéant, à l'aide d'un ou de plusieurs radicaux C₁-C₁₅-alkyles, qui est interrompu, le cas échéant, par de 1 à 4 atomes d'oxygène en formant une fonction éther, un radical C₁-C₁₅-alkoxy, C₁-C₁₅-alkylamino ou C₁-C₁₅-dialkylamino,
ou
un radical hétéroaryle-C₁-C₄-alkyle ayant de 3 à 5 atomes de carbone dans le radical hétérocyclique, ce dernier étant substitué, le cas échéant, à l'aide d'un ou de plusieurs radicaux C₁-C₁₅-alkyle, qui est interrompu, le cas échéant, par de 1 à 4 atomes d'oxygène en formant une fonction éther, un radical C₁-C₁₅-alkoxy, C₁-C₁₅-alkylamino ou C₁-C₁₅-dialkylamino,
_{R}² désigne l'hydrogène ou indépendamment de R¹, a la signification de R¹, au cas où X² correspond à un groupement carbonyle, R² ne possède pas la signification d'hydrogène,
et
n désigne, indépendamment les unes des autres, des valeurs de 0, 1, ou 2, sous réserve que la somme des quatre valeurs de n soit d'au moins 1.

7. Utilisation selon la revendication 1, **caractérisée en ce que**, dans la formule I,
M désigne deux fois l'hydrogène,
R désigne des groupements -CH₂-N(-X¹-R¹) (X²-R²) identiques ou différents les uns des autres,
X¹, X² désignent, indépendamment l'un de l'autre, un groupement carbonyle ou une liaison chimique simple,
R¹ désigne un radical C₁-C₁₅,-alkyle, qui est interrompu, le cas échéant, par de 1 à 4 atomes d'oxygène en formant une fonction éther,
un radical cyclohexyle, qui est substitué, le cas échéant, à l'aide d'un ou de plusieurs groupements C₁-C₁₅-alkyles, qui sont, le cas échéant, interrompus par de 1 à 4 atomes d'oxygène en formant une fonction éther,
un radical hétérocyclique saturé, à cinq ou six membres, qui est substitué, le cas échéant, à l'aide d'un ou de plusieurs groupements C₁-C₁₅-alkyles, qui sont, le cas échéant, interrompus par de 1 à 4 atomes d'oxygène en formant une fonction éther,
un radical phényle, qui est substitué, le cas échéant, à l'aide d'un ou de plusieurs radicaux C₁-C₁₅-alkyles, qui est interrompu, le cas échéant, par de 1 à 4 atomes d'oxygène en formant une fonction éther, un radical C₁-C₁₅-alkoxy, C₁-C₁₅-alkylamino ou C₁-C₁₅-dialkylamino,
ou
un radical phényle-C₁-C₄-alkyle, qui est substitué, dans le radical aryle, le cas échéant, à l'aide d'un ou de plusieurs radicaux C₁-C₁₅-alkyles, qui est interrompu, le cas échéant, par de 1 à 4 atomes d'oxygène en formant une fonction éther, un radical C₁-C₁₅-alkoxy, C₁-C₁₅-alkylamino ou C₁-C₁₅-dialkylamino,
R² désigne l'hydrogène ou indépendamment de R¹, a la signification de R¹, au cas où X² correspond à un groupement carbonyle, R² ne possède pas la signification d'hydrogène,
et
n désigne, indépendamment les unes des autres, des valeurs de 0, 1, ou 2, sous réserve que la somme des quatre valeurs de n soit d'au moins 1.

8. Utilisation de phtalocyanines de la formule I selon l'une quelconque des revendications 1 à 7, en tant que substances de marquage pour les huiles minérales.

9. Fluides contenant au moins une phtalocyanine de la formule I, selon l'une quelconque des revendications 1 à 7 en tant que substance de marquage.

10. Huiles minérales contenant au moins une phtalocyanine de la formule I selon l'une quelconque des revendications 1 à 7 en tant que substance de marquage.

11. Phtalocyanines de la formule Ia 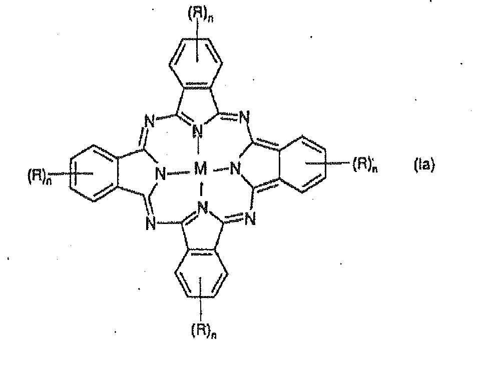 étant donné que, dans la formule Ia,
M désigne deux fois l'hydrogène,
R désigne des groupements -CH₂-Het, identiques ou différents les uns des autres,
Het signifie un radical saturé, contenant de l'azote, à cinq, six ou sept membres, qui est substitué, le cas échéant, à l'aide d'un ou de plusieurs groupements C₁-C₄-alkyles, et dans lequel, le cas échéant, un groupement CH₂ est remplacé par un groupement carbonyle,
la liaison du radical hétérocyclique saturé au groupement CH₂ du groupement -CH₂-Het se faisant ou par un atome d'azote approprié ou par un atome de carbone,
et
n désigne, indépendamment les unes des autres, des valeurs de 0, 1, ou 2, sous réserve que la somme des quatre valeurs de n soit d'au moins 1.

12. Phtalocyanines selon la revendication 11, étant donné que, dans la formule Ia,
M désigne deux fois l'hydrogène,
R désigne des groupements -CH₂-Het identiques,
Het désigne un radical γ-butyrolactame, δ-valérolactame ou ε-caprolactame, qui est substitué, le cas échéant, à l'aide d'un ou de plusieurs groupements C₁-C₄-alkyles et auquel le groupement CH₂ du groupement -CH₂-Het est lié à l'aide de l'atome d'azote
et
n désigne, indépendamment les unes des autres, des valeurs de 0, 1, ou 2, sous réserve que la somme des quatre valeurs de n soit d'au moins 1.
